# FASCICULE DE BREVET EUROPEEN

(11) **EP 1 525 217 B1**
(45) Date de publication et mention de la délivrance du brevet: **12.05.2010**
(21) Numéro de dépôt: 03762736.1
(22) Date de dépôt: 04.07.2003
(51) Int. Cl.: C07K 7/08, C07K 14/415, C12N 15/29, C12N 15/82, C07K 16/16, A01H 5/00, A01N 43/38, A01N 37/18

(54) **PEPTIDE DE PLANTE A ACTIVITE ANTI-MICROBIENNE**
PEPTID AUS PFLANZEN MIT ANTIMIKROBIELLER AKTIVITÄT
PLANT PEPTIDE WITH ANTIMICROBIAL ACTIVITY

(30) Priorité: 05.07.2002 FR 0208447
(43) Date de publication de la demande: 27.04.2005
(73) Titulaire: CENTRE NATIONAL DE LA RECHERCHE SCIENTIFIQUE (CNRS), 75794 Paris Cedex 16 (FR)
(72) Inventeur: GALLOIS, Patrick, SALE, Cheshire M33 2 DE (GB); HECHT, Valérie, ANDORRA LA VELLA (AD); VAROQUAUX, Fabrice, F-66650 BANYULS sur MER (FR); BLANVILLAIN, Robert, BERKELEY, CA 94708 (US); PUERTOLAS, Delphine, F-31520 RAMONVILLE (FR); DELORME, Valérie, F-66000 PERPIGNAN (FR); ROBY, Dominique, F-31450 POMPERTUZAT (FR); DELSENY, Michel, F-66430 BOMPAS (FR)
(74) Mandataire: Vialle-Presles, Marie José
(86) Numéro de dépôt international: PCT/FR2003/002077
(87) Numéro de publication internationale: WO 2004/005329

(56) Documents cités:
- WO-A-01/71042
- WO-A-93/05153
- US-A- 4 613 355
- DATABASE EMBL [en ligne] 11 janvier 1999 (1999-01-11) LAPLANT Y & SPALDING L.: "Arabidopsis thaliana BAC F3H7" retrieved from EBI Database accession no. AF118222 XP002235082
- DATABASE EMBL [en ligne] 3 juin 1999 (1999-06-03) GALLOIS P ET AL.: "Arabidopsis thaliana RNA-binding protein (RBP37) gene" retrieved from EBI Database accession no. AF109721 XP002235081
- DATABASE EMBL [Online] 25 March 1999 (1999-03-25), retrieved from EBI Database accession no. AL049523
- DATABASE EMBL [Online] 16 March 2000 (2000-03-16), retrieved from EBI Database accession no. AL161517

### SEQUENCE LISTING

<110> Centre National de la Recherche Scientifique
   GALLOIS Patrick
   HECHT Valérie
   VAROQUAUX Fabrice
   BLANVILLAIN Robert
   PUERTOLAS Delphine
   DELORME Valérie
   ROBY Dominique
   DELSENY Michel
<120> Nouveau peptide de plante à activité anti-microbienne
<130> CGA644s70
<160> 31
<170> PatentIn version 3.1
<210> 1
   <211> 16
   <212> PRT
   <213> Arabidopsis thaliana
<220>
   <221> MISC_FEATURE
   <222> (16)..(16)
   <223> Xaa = cysteine ou serine
<400> 1
   Met Trp Trp Leu Val Gly Leu Thr Pro Val Glu Leu Ile His Leu Xaa
   1 5 10 15
<210> 2
   <211> 17
   <212> PRT
   <213> Arabidopsis thaliana
<220>
   <221> MISC_FEATURE
   <222> (16)..(16)
   <223> Xaa = cysteine or serine
<400> 2
   Met Trp Trp Leu Val Gly Leu Thr Pro Val Glu Leu Ile His Leu Xaa
   1 5 10 15
   Ala
<210> 3
   <211> 18
   <212> PRT
   <213> Arabidopsis thaliana
<220>
   <221> MISC_FEATURE
   <222> (16).. (16)
   <223> Xaa = cysteine or histidine
<400> 3
   Met Trp Trp Leu Val Gly Leu Thr Pro Val Glu Leu Ile His Leu Xaa
   1 5 10 15
   Ala Phe
<210> 4
   <211> 19
   <212> PRT
   <213> Arabidopsis thaliana
<220>
   <221> MISC_FEATURE
   <222> (16)..(16)
   <223> X= cysteine or serine
<220>
   <221> MISC_FEATURE
   <222> (16)..(16)
   <223> Xaa= cysteine or serine
<400> 4 Ala Phe Arg
<210> 5
   <211> 20
   <212> PRT
   <213> Arabidopsis thaliana
<220>
   <221> MISC_FEATURE
   <222> (16) .. (16)
   <223> Xaa = cysteine or serine
<400> 5
   Met Trp Trp Leu Val Gly Leu Thr Pro Val Glu Leu Ile His Leu Xaa
   1 5 10 15
   Ala Phe Arg Glu
   20
<210> 6
   <211> 21
   <212> PRT
   <213> Arabidopsis thaliana
<220>
   <221> MISC_FEATURE
   <222> (16).. (16)
   <223> Xaa = cysteine or serine
<400> 6
   Met Trp Trp Leu Val Gly Leu Thr Pro Val Glu Leu Ile His Leu Xaa
   1 5 10 15
   Ala Phe Arg Glu Arg
   20
<210> 7
   <211> 22
   <212> PRT
   <213> Arabidopsis thaliana
<220>
   <221> MISC_FEATURE
   <222> (16)..(16)
   <223> Xaa = cysteine or serine
<400> 7
   Met Trp Trp Leu Val Gly Leu Thr Pro Val Glu Leu Ile His Leu Xaa
   1               5                   10                  15
   Ala Phe Arg Glu Arg Leu
               20
<210> 8
   <211> 23
   <212> PRT
   <213> Arabidopsis thaliana
<220>
   <221> MISC_FEATURE
   <222> (16)..(16)
   <223> Xaa = cysteine or histidine
<220>
   <221> MISC_FEATURE
   <222> (23)..(23)
   <223> Xaa = cysteine or histidine
<400> 8
   Met Trp Trp Leu Val Gly Leu Thr Pro Val Glu Leu Ile His Leu Xaa
   1               5                   10                  15
   Ala Phe Arg Glu Arg Leu Xaa
               20
<210> 9
   <211> 24
   <212> PRT
   <213> Arabidopsis thaliana
<220>
   <221> MISC_FEATURE
   <222> (16)..(16)
   <223> Xaa = cysteine or serine
<220>
   <221> MISC_FEATURE
   <222> (23)..(23)
   <223> Xaa = cysteine or serine
<400> 9
   Met Trp Trp Leu Val Gly Leu Thr Pro Val Glu Leu Ile His Leu Xaa
   1               5                   10                  15
   Ala Phe Arg Glu Arg Leu Xaa His
               20
<210> 10
   <211> 25
   <212> PRT
   <213> Arabidopsis thaliana
<220>
   <221> MISC_FEATURE
   <222> (16)..(16)
   <223> Xaa= cysteine or histidine
<220>
   <221> MISC_FEATURE
   <222> (23)..(23)
   <223> Xaa= cysteine or histidine
<400> 10
   Met Trp Trp Leu Val Gly Leu Thr Pro Val Glu Leu Ile His Leu Xaa
   1               5                   10                  15
   Ala Phe Arg Glu Arg Leu Xaa His Leu
               20                  25
<210> 11
   <211> 48
   <212> DNA
   <213> Arabidopsis thaliana
<220>
   <221> misc_feature
   <222> (46)..(48)
   <223> nnn = codon cystéine ou codon srine
<400> 11
   atgtggtggc tagttggact tacaccagtt gagttgatcc atcttnnn 48
<210> 12
   <211> 51
   <212> DNA
   <213> Arabidopsis thaliana
<220>
   <221> misc_feature
   <222> (46)..(48)
   <223> nnn = codon cystéine ou codon sérine
<400> 12
   atgtggtggc tagttggact tacaccagtt gagttgatcc atcttnnngc a 51
<210> 13
   <211> 54
   <212> DNA
   <213> Arabidopsis thaliana
<220>
   <221> misc_feature
   <222> (46)..(48)
   <223> nnn = codon cystéine ou codon sérine
<400> 13
   atgtggtggc tagttggact tacaccagtt gagttgatcc atcttnnngc attt 54
<210> 14
   <211> 57
   <212> DNA
   <213> Arabidopsis thaliana
<220>
   <221> misc_feature
   <222> (46)..(48)
   <223> nnn = codon cystéine ou codon sérine
<400> 14
   atgtggtggc tagttggact tacaccagtt gagttgatcc atcttnnngc atttcga 57
<210> 15
   <211> 60
   <212> DNA
   <213> Arabidopsis thaliana
<220>
   <221> misc_feature
   <222> (46) .. (48)
   <223> nnn = codon cystéine ou codon sérine
<400> 15
   atgtggtggc tagttggact tacaccagtt gagttgatcc atcttnnngc atttcgagag 60
<210> 16
   <211> 63
   <212> DNA
   <213> Arabidopsis thaliana
<220>
   <221> misc_feature
   <222> (46) .. (48)
   <223> nnn = codon cystéine ou codon sérine
<400> 16
   atgtggtggc tagttggact tacaccagtt gagttgatcc atcttnnngc atttcgagag 60
   cgt 63
<210> 17
   <211> 66
   <212> DNA
   <213> Arabidopsis thaliana
<220>
   <221> misc_feature
   <222> (46)..(48)
   <223> nnn = codon cystéine ou codon sérine
<400> 17
   atgtggtggc tagttggact tacaccagtt gagttgatcc atcttnnngc atttcgagag 60
   cgtctc 66
<210> 18
   <211> 69
   <212> DNA
   <213> Arabidopsis thaliana
<220>
   <221> misc_feature
   <222> (46)..(98)
   <223> nnn = codon cystéine ou codon sérine
<220>
   <221> misc_feature
   <222> (67)..(69)
   <223> nnn = codon cystéine ou codon sérine
<400> 18
   atgtggtggc tagttggact tacaccagtt gagttgatcc atcttnnngc atttcgagag 60
   cgtctcnnn 69
<210> 19
   <211> 72
   <212> DNA
   <213> Arabidopsis thaliana
<220>
   <221> misc_feature
   <222> (46).. (48)
   <223> nnn = codon cystéine ou codon sérine
<220>
   <221> misc_feature
   <222> (67)..(69)
   <223> nnn = codon cystéine ou codon sérine
<400> 19
   atgtggtggc tagttggact tacaccagtt gagttgatcc atcttnnngc atttcgagag 60
   cgtctcnnnc at 72
<210> 20
   <211> 75
   <212> DNA
   <213> Arabidopsis thaliana
<220>
   <221> misc_feature
   <222> (46)..(48)
   <223> nnn = codon cystéine ou codon sérine
<220>
   <221> misc_feature
   <222> (67)..(69)
   <223> nnn = codon cystéine ou codon sérine
<400> 20
   atgtggtggc tagttggact tacaccagtt gagttgatcc atcttnnngc atttcgagag 60
   cgtctcnnnc atctc 75
<210> 21
   <211> 20
   <212> DNA
   <213> Artificial sequence
<220>
   <223> Amorce
<400> 21
   caagtaagct tgctcagttg 20
<210> 22
   <211> 29
   <212> DNA
   <213> Artificial sequence
<220>
   <223> Amorce
<400> 22
   gctctagata cttagagatg acagagacg 29
<210> 23
   <211> 30
   <212> DNA
   <213> Artificial sequence
<220>
   <223> Amorce
<400> 23
   actgagctcg ttgtattttt aatcgaatgg 30
<210> 24
   <211> 31
   <212> DNA
   <213> Artificial sequence
<220>
   <223> Amorce
<400> 24
   acggaagctt tacttagaga tgacagagac g 31
<210> 25
   <211> 21
   <212> DNA
   <213> Artificial sequence
<220>
   <223> Amorce
<400> 25
   cgtcttcgag aaaagtgtta g 21
<210> 26
   <211> 21
   <212> DNA
   <213> Artificial sequence
<220>
   <223> Amorce
<400> 26
   atcagtcaga cagtcaaatt c 21
<210> 27
   <211> 21
   <212> DNA
   <213> Artificial sequence
<220>
   <223> Amorce
<400> 27
   tacttagaga tgacagagac g 21
<210> 28
   <211> 86
   <212> DNA
   <213> Artificial sequence
<220>
   <223> Oligonucléotide complémentaire de SEQ ID NO : 29
<400> 28
   gatcatgtgg tggctagttg gacttacacc agttgagttg atccatcttt gcgcatttcg 60
   agagcgtctc tgtcatctct aatgca 86
<210> 29
   <211> 78
   <212> DNA
   <213> Artificial sequence
<220>
   <223> Oligonucléotide complémentaire de SEQ ID NO : 28
<400> 29
   ttagagatga cagagacgct ctcgaaatgc gcaaagatgg atcaactcaa ctggtgtaag 60
   tccaactagc caccacat 78
<210> 30
   <211> 20
   <212> DNA
   <213> Artificial Sequence
<220>
   <223> Amorce
<400> 30
   cttcgtcaac atggtggagc 20
<210> 31
   <211> 19
   <212> DNA
   <213> Artificial Sequence
<220>
   <223> Amorce
<400> 31
   ctagctagag gatcgatcc 19

## Description

La présente invention est relative à de nouveaux peptides isolés, naturels ou synthétiques, à activité anti-microbienne, aux polynucléotides codant pour lesdits peptides, aux vecteurs comprenant lesdits polynucléotides, aux microorganismes et aux cellules transformées avec lesdits vecteurs, aux organismes transgéniques non humains dont tout ou partie des cellules contiennent et/ou expriment lesdits vecteurs, aux utilisations desdits peptides et desdits polynucléotides, particulièrement comme agents anti-microbiens spécifiques des plantes, ainsi qu'à un procédé de traitement anti-microbien des plantes.

Des peptides issus de plantes à activité anti-microbienne, sont connus dans l'art antérieur. A cet égard, on peut citer les peptides à activité lytique tels que les défensines, les cécropines, les thionines, les mellitines, issues de mammifères ou encore les défensines, les magainines, les attacines, les dipterines, les sapecines, les caerulines, les xenopsines, isolées à partir d'insectes. Des hybrides de ces peptides ont également été décrits.

Des peptides de lapins ont également été décrits pour leur activité anti-microbienne, ainsi que des peptides à activité hydrolytique comme la chitinase ou la β-1,3-glucanase.

Parmi les peptides issus de plantes, on peut citer ceux décrits dans les brevets WO 93/05153, US 6,147,281, US 6,150,588 ou encore US 5,424,395.

La demande WO 93/05153 décrit des protéines isolées à partir de graines de plantes des familles des brassicacées, des composées et des légumineuses, présentant une activité antifongique et, pour certaines, une activité antibactérienne contre les bactéries Gram-positives.

Le brevet US 6,147,281 décrit des peptides ayant une activité anti-microbienne sur les pathogènes de plantes, présents dans des feuilles étiolées d'orge. Ces peptides présentent particulièrement une activité contre *Corynebacterium sepedonicum.*

Le brevet US 6,150,588 décrit des peptides ayant une activité anti-microbienne sur les pathogènes de plantes et pouvant être isolés à partir de graines d'Aralia ou d'impatiens.

Le brevet US 5,424,395 décrit des peptides dérivés de la magainine 2 ayant une activité anti-microbienne sur les pathogènes de plantes.

Les peptides normalement présents dans les plantes et présentant une activité anti-microbienne constituent une classe d'agents de lutte biologique contre les agents pathogènes particulièrement intéressante. L'identification de nouveaux composés dérivés de plantes, susceptibles de présenter une activité anti-microbienne spécifique vis-à-vis des plantes permet d'envisager le développement de nouveaux biopesticides qui constituent un réservoir de produits en cas d'apparition de résistance. C'est dans ce cadre que se place la présente invention.

De manière surprenante et inattendue, les Inventeurs ont isolé dans *Arabidopsis thaliana* un peptide qui présente la particularité d'avoir une activité anti-microbienne, particulièrement contre les agents pathogènes des plantes. Dans son activité anti-microbienne, ledit peptide présente une activité au moins bactériostatique, éventuellement bactéricide. Très particulièrement, ledit peptide présente une activité anti-microbienne spécifique de certaines espèces bactériennes, particulièrement d'espèces pathogènes des plantes, comme par exemple *Xanthomonas campestris, Pseudomonas syringae* ou encore *Erwinia amylovora.*

L'activité anti-microbienne peut se manifester sous la forme de deux actions distinctes : inhibition "microbicide" (bactéricide, virucide ou fongicide), qui consiste à tuer lesdits microorganismes ; et inhibition "microbiostatique" (bactériostatique, virostatique ou fongistatique) qui consiste à réduire ou à inhiber les capacités de prolifération desdits microorganismes.

En conséquence, l'invention a pour objet un peptide isolé, naturel ou synthétique, comprenant au moins l'une quelconque des séquences SEQ ID NO : 1 à SEQ ID NO : 10 suivantes :
SEQ ID N° 1 : Met-Trp-Trp-Leu-Val-Gly-Leu-Thr-Pro-Val-Glu-Leu-lleu-His-Leu-R₁
SEQ ID N° 2 : Met-Trp-Trp-Leu-Val-Gly-Leu-Thr-Pro-Val-Glu-Leu-lleu-His-Leu-R₁-Ala
SEQ ID N° 3 : Met-Trp-Trp-Leu-Val-Gly-Leu-Thr-Pro-Val-Glu-Leu-lleu-His-Leu-R₁-Ala-Phe
SEQ ID N° 4 : Met-Trp-Trp-Leu-Val-Gly-Leu-Thr-Pro-Val-Glu-Leu-lleu-His-Leu-R₁-Ala-Phe-Arg
SEQ ID N° 5 : Met-Trp-Trp-Leu-Val-Gly-Leu-Thr-Pro-Val-Glu-Leu-lleu-His-Leu-R₁-Ala-Phe-Arg-Glu
SEQ ID N° 6 : Met-Trp-Trp-Leu-Val-Gly-Leu-Thr-Pro-Val-Glu-Leu-lieu-His-Leu-R₁-Ala-Phe-Arg-Glu-Arg
SEQ ID N° 7 : Met-Trp-Trp-Leu-Val-Gly-Leu-Thr-Pro-Val-Glu-Leu-Ileu-His-Leu-R₁-Ala-Phe-Arg-Glu-Arg-Leu
SEQ ID N° 8 : Met-Trp-Trp-Leu-Val-Gly-Leu-Thr-Pro-Val-Glu-Leu-lleu-His-Leu-R₁-Ala-Phe-Arg-Glu-Arg-Leu-R₂
SEQ ID N° 9 : Met-Trp-Trp-Leu-Val-Gly-Leu-Thr-Pro-Val-Glu-Leu-lleu-His-Leu-R₁-Ala-Phe-Arg-Glu-Arg-Leu-R₂-His
SEQ ID N° 10 : Met-Trp-Trp-Leu-Val-Gly-Leu-Thr-Pro-Val-Glu-Leu-lleu-His-Leu-R₁-Ala-Phe-Arg-Glu-Arg-Leu-R₂-His-Leu
dans lesquelles R₁ et R₂ représentent, indépendamment ou simultanément, une cystéine ou une sérine.

Le peptide selon l'invention comprend au moins la séquence SEQ ID NO : 1.

Selon un mode de réalisation avantageux de l'invention, ledit peptide consiste en l'une quelconque des séquences SEQ ID NO : 1 à SEQ ID NO : 10, telles que définies ci-dessus.

Par isolé, on entend ici tout peptide de quelque origine qu'il soit, qui aura, à partir de sa source, subit au moins une étape d'enrichissement. Ainsi, l'invention couvre aussi bien des extraits bruts, par exemple de végétaux ou de cellules végétales, contenant au moins un peptide selon l'invention, que des formes de celui-ci beaucoup plus pures.

De manière avantageuse, le peptide selon l'invention peut être soit isolé de plantes (*Arabidopsis thaliana* par exemple) soit obtenu par synthèse chimique ou encore par des moyens biotechnologiques comme par exemple à partir de microorganismes, de cellules de plantes ou d'animaux, voire d'organismes modifiés, qui n'expriment normalement pas ledit peptide.

L'invention a également pour objet un peptide isolé, dont la séquence est substantiellement homologue à l'une au moins des séquences SEQ ID NO : 1 à SEQ ID NO : 10, telles que définies ci-dessus.

On considère ici qu'un peptide présente une séquence substantiellement homologue lorsque sa séquence en acides aminés présente au moins 60% de similarité avec la séquence en acides aminés d'au moins l'une des séquences SEQ ID NO : 1 à SEQ ID NO : 10 et que le peptide a conservé son activité initiale anti-microbienne, particulièrement son activité spécifique vis-à-vis de microorganismes pathogènes de plantes.

Par 60% de similarité entre un peptide P et les séquences SEQ ID NO : 1 à 10, on entend que lorsque les deux peptides sont alignés, 60% des acides aminés de P sont identiques à l'acide aminé correspondant des séquences SEQ ID NO : 1 à 10, ou sont remplacés par un acide aminé du même groupe.

Par acide aminé de même groupe, on entend un acide aminé possédant des propriétés chimiques sensiblement identiques. En particulier, on entend par ce terme des acides aminés ayant sensiblement la même charge et/ou la même taille, et/ou la même hydrophilie ou hydrophobie et/ou la même aromaticité.

De tels groupes d'acides aminés incluent notamment ;
(i) glycine, alanine
(ii) isoleucine, leucine, valine
(iii) tryptophane, tyrosine, phénylalanine
(iv) acide aspartique, acide glutamique
(v) arginine, lysine, histidine
(vi) sérine, thréonine

D'autres substitutions peuvent être envisagées, dans lesquelles on remplace un acide aminé par un autre acide aminé comparable mais non naturel (hydroxyproline, norleucine, ornithine, citrulline, cyclohexylalanine, acides aminés dextrogyres, ....)

Le fait que le peptide selon l'invention présente les propriétés anti-microbiennes précédemment décrites permet d'envisager son utilisation comme agent anti-microbien, particulièrement dirigé contre les agents pathogènes de plantes. Très particulièrement, le peptide selon l'invention peut être utilisé comme agent bactériostatique, éventuellement bactéricide, encore plus particulièrement comme agent anti-microbien spécifique de *Xanthomonas campestris,* de *Pseudomonas syringae* ou encore *d'Erwinia amylovora.* Cela permet d'envisager son utilisation comme biopesticide, ne présentant pas d'effets secondaires toxiques sur l'environnement.

L'invention a également pour objet l'utilisation d'un peptide, isolé, comprenant ou consistant en au moins l'une quelconque des séquences SEQ ID NO : 1 à SEQ ID NO : 10, comme agent anti-microbien, particulièrement dirigé contre les agents pathogènes de plantes.

Selon un mode de réalisation avantageux de ladite utilisation, ledit peptide selon l'invention est un agent bactériostatique, éventuellement bactéricide, et encore plus particulièrement un agent anti-microbien spécifique de *Xanthomonas campestris,* de *Pseudomonas syringae* ou d'*Erwinia amylovora.*

De manière surprenante, les Inventeurs ont montré que :
- les peptides selon l'invention comprenant ou consistant en au moins l'une quelconque des séquences SEQ ID NO : 1 à SEQ ID NO : 10, dans lesquelles R₁ représente une sérine, conservent leur activité anti-microbienne ;
- les peptides selon l'invention comprenant ou consistant en au moins l'une quelconque des séquences SEQ ID NO : 2 à SEQ ID NO : 10, dans lesquelles R₁ représente une cystéine ou une sérine et R₂ représente une cystéine, présentent une activité cytotoxique ;
- les peptides selon l'invention comprenant ou consistant en au moins l'une quelconque des séquences SEQ ID NO : 8 à 10, dans lesquelles R₁ représente une cystéine et R₂ représente une sérine, présentent une activité cytotoxique fortement réduite, voire inhibée, alors que l'activité anti-microbienne est conservée ;
- les peptides selon l'invention comprenant ou consistant en au moins l'une quelconque des séquences SEQ ID NO : 8 à 10, dans lesquelles R₁ et R₂ représentent simultanément une sérine, présentent une activité cytotoxique.

L'activité cytotoxique du peptide selon l'invention est perdue lorsque le peptide comprend ou consiste uniquement en la séquence SEQ ID NO:1.

En conséquence, la présente invention a également pour objet l'utilisation d'au moins un peptide comprenant ou consistant en au moins l'une des séquences SEQ ID NO : 2 à SEQ ID NO : 10, dans lesquelles R₁ représente une cystéine ou une sérine et R₂ représente une cystéine, ou lorsqu'il comprend ou qu'il consiste en au moins l'une des séquences SEQ ID NO : 8 à SEQ ID NO : 10, dans lesquelles R₁ et R₂ représentent simultanément une sérine, comme agent cytotoxique, particulièrement comme agent cytotoxique pour les cellules végétales.

La présente invention a également pour objet l'utilisation du peptide selon l'invention comprenant ou consistant en au moins l'une des séquences SEQ ID NO : 2 à SEQ ID NO : 10, dans lesquelles R₁ représente une cystéine ou une sérine et R₂ représente une cystéine, ou lorsqu'il comprend ou qu'il consiste en au moins l'une des séquences SEQ ID NO : 8 à SEQ ID NO : 10, dans lesquelles R₁ et R₂ représentent simultanément une sérine, comme agent désherbant.

L'invention a aussi pour objet l'utilisation du peptide selon l'invention lorsqu'il comprend ou qu'il consiste en au moins l'une des séquences SEQ ID NO : 8 à SEQ ID NO : 10, dans lesquelles R₁ représente une cystéine et R₂ représente une sérine, comme agent anti-microbien non cytotoxique, particulièrement dirigé contre les agents pathogènes de plantes.

De manière avantageuse, les fonctions carboxyles et amines libres des peptides selon l'invention peuvent être protégées.

Les groupements protecteurs usuels sont bien connus. Par exemple, le peptide selon l'invention peut être un peptide pour lequel le groupement carboxylique C-terminal, et éventuellement les autres groupements carboxyliques présents dans la molécule, sont sous la forme d'un ester (ester d'alkyle inférieur ayant de 1 à 4 carbones) ou d'un amide, et/ou celui pour lequel le groupement amine N-terminal, et éventuellement les autres groupements amines présents dans la molécule, sont sous forme acylée (par exemple acétylée). Plus généralement, l'invention inclut non seulement les sels d'addition du peptide avec des sels organiques carboxyliques et les acétates, mais aussi d'autres sels d'addition tels que par exemple les trifluoroacétates, ainsi que les sels d'addition du peptide avec les acides minéraux tels que les sulfates, les chlorhydrates, etc. L'invention inclut également les sels résultant de la salification du (ou des) groupe carboxylique, et notamment les sels des métaux alcalins ou alcalino-terreux tels que les sels de sodium ou de calcium.

L'invention a également pour objet une composition comprenant au moins un peptide comprenant ou consistant en au moins l'une quelconque des séquences SEQ ID NO : 1 à SEQ ID NO : 10, telles que décrites précédemment et au moins un véhicule approprié.

La composition selon l'invention peut être une compositions à usage alimentaire, pharmaceutique, vétérinaire (composition anti-microbienne ou conservatrice) ou bien entendu encore une composition à usage agricole (biopesticide et/ou désherbant).

L'invention a également pour objet un polynucléotide isolé, naturel ou synthétique, caractérisé en ce qu'il consiste en au moins une séquence codant pour l'un au moins des peptides selon l'invention.

Selon un mode de réalisation avantageux dudit polynucléotide, il comprend au moins l'une des séquences SEQ ID NO : 11 à SEQ ID NO : 20 suivantes : dans laquelle R₃ et R₄ représentent, indépendamment ou simultanément, un codon cystéine ou un codon sérine.

Selon une disposition avantageuse de ce mode de réalisation, le polynucléotide selon l'invention consiste en l'une des séquences SEQ ID NO : 11 à SEQ ID NO : 20, dans laquelle R₃ et R₄ représentent un codon cystéine ou un codon sérine.

Le polynucléotide selon l'invention peut être sous la forme d'acide désoxyribonucléique (ADN) ou d'acide ribonucléique (ARN), simple brin ou double brin.

Le polynucléotide selon l'invention peut être déduit de la séquence de l'un quelconque des peptides selon l'invention et synthétisé à l'aide d'un synthétiseur à ADN. Le polynucléotide peut également être obtenu à partir de banques d'ADN, particulièrement de banques d'ADN de cellules végétales, très particulièrement à partir d'une banque d'ADN de cellules *d'Arabidopsis thaliana.*

L'invention a également pour objet une composition comprenant, dans un milieu approprié, au moins un polynucléotide, tel que décrit précédemment, et au moins un véhicule approprié.

Pour l'expression desdits peptides selon l'invention, les polynucléotides peuvent avantageusement être introduits dans un vecteur approprié.

Le vecteur utilisé peut être tout vecteur connu de l'art antérieur. Particulièrement, on peut citer comme vecteurs utilisables selon l'invention les plasmides, les virus ou encore les bactériophages.

Parmi les plasmides utilisables selon l'invention, on peut citer ceux décrits par R. L. Rodriguez et D. T. Denhardt (A Survey of Molecular Cloning Vectors and Their Uses, Butterworths, Boston, 1998) ou encore par J. Sambrook et al., (Molecular cloning, Cold Spring Arbor, 2001)

Particulièrement, les vecteurs pMOSBlue®, vendus par la société Amersham, les vecteurs pGEM®-T et pGEM®-T easy vendus par la société Promega ou encore les plasmides du type *Agrobacterium* Ti (H. De Greve et al., J. Mol.. Appl. Genet., 1, (1982), 499-511) peuvent avantageusement être utilisés.

Ledit vecteur peut comporter en outre toutes séquences régulatrices requises pour la réplication du vecteur et/ou l'expression du peptide codé par le polynucléotide (promoteur, sites de terminaison, etc).

Dans une construction particulièrement intéressante, le polynucléotide selon l'invention est placé sous la dépendance d'un promoteur inductible, rendant ainsi la synthèse du peptide dépendante de conditions prédéfinies, par exemple de conditions environnementales, de la présence d'un agent pathogène ou d'un agent chimique.

L'invention a donc aussi pour objet un vecteur comprenant l'un quelconque des polynucléotides tels que décrits précédemment, comprenant ou consistant en l'une au moins des SEQ ID NO : 11 à SEQ ID NO:20 dans lesquelles R₃ et R₄ représentent, indépendamment ou simultanément, un codon cystéine ou un codon sérine.

L'invention a en outre pour objet l'utilisation d'un polynucléotide ou d'un vecteur tels que décrits précédemment pour la préparation d'un peptide selon l'invention.

L'invention a aussi pour objet un système biologique modifié dans lequel, au moins un polynucléotide selon l'invention ou au moins un vecteur de l'invention a été introduit.

Un tel système biologique peut être par exemple un microorganisme, comme une bactérie telle que *Escherichia coli,* un endophyte comme ceux décrit dans les Demandes Internationales WO90/13224 WO91/10363, WO87/03303, WO94/16076 ou encore la Demande de Brevet EP-125468 ou encore une levure telle que *Saccharomyces cerevisiae,* une cellule comme par exemple une cellule d'insecte, une cellule animale ou une cellule végétale.

L'invention a également pour objet l'utilisation du polynucléotide selon l'invention et/ou du vecteur de l'invention pour la préparation d'un système biologique modifié, celui-ci pouvant être un microorganisme, un endophyte, une levure ou une cellule eucaryote.

L'invention a aussi pour objet une composition comprenant au moins un système biologique tel que décrit précédemment et au moins un véhicule approprié.

L'invention a aussi pour objet l'utilisation d'au moins un système biologique modifié tel que décrit précédemment ou d'une composition comprenant au moins un système biologique modifié telle que décrite précédemment pour la préparation d'une composition agent anti-microbienne, particulièrement dirigée contre les agents pathogènes de plantes, et/ou d'une composition cytotoxique.

Le système biologique modifié peut éventuellement en outre permettre la sécrétion du peptide selon l'invention dans un milieu de culture rendant son extraction et sa purification plus faciles.

L'introduction du polynucléotide et/ou du vecteur selon l'invention dans le système biologique modifié hôte, peut se faire par toute méthode connue, comme par exemple la transfection, l'infection, la fusion, l'électroporation, la microinjection ou encore la biolistique.

L'invention a encore pour objet un organisme transgénique non humain dont tout ou partie des cellules contient le polynucléotide selon l'invention ou le vecteur de l'invention, sous une forme libre ou intégrée.

Particulièrement, l'organisme transgénique de l'invention est un végétal transgénique.

Le végétal transgénique peut appartenir à toute espèce végétale, particulièrement à une espèce végétale susceptible d'être infectée par *Xanthomonas campestris* et/ou *Pseudomonas syringae* et/ou *Erwinia amylovora.*

On peut citer à titre d'exemple pour *Xanthomonas campestris,* le chou, la luzerne, le soja, le coton, le pois ou encore les céréales, pour *Erwinia amylovora,* le poirier, le pommier ou encore la pomme de terre et pour *Pseudomonas syringae,* la banane, le blé ou encore les végétaux de la famille des Citrus.

L'organisme transgénique de l'invention, particulièrement le végétal transgénique, lorsqu'il exprime le peptide selon l'invention, présente une résistance améliorée aux pathogènes, particulièrement aux phytopathogènes, très particulièrement à *Xanthomonas campestris* et/ou *Pseudomonas syringae* et/ou *Erwinia amylovora.*

L'invention a donc aussi pour objet un organisme transgénique non humain, particulièrement un végétal transgénique présentant une résistance améliorée aux pathogènes, particulièrement aux phytopathogènes, très particulièrement à *Xanthomonas campestris* et/ou *Pseudomonas syringae* et/ou *Erwinia amylovora.*

Toute méthode de préparation d'organisme transgénique, particulièrement les méthodes appliquées aux végétaux peuvent être utilisées pour la préparation des organismes transgéniques de l'invention.

A cet égard on peut citer les méthodes décrites dans Plant gene Transfer and Expression Protocols - Methods in Molecular Biology (Humana Press, H. Jones éditeur, 1995, 49 : 39-48) ou encore celles décrites par P. Gallois et al., dans Plant Cell Electroporation and Electrofusion Protocols - Methods in Molecular Biology, (Humana Press, J.A. Nickoloff éditeur, 1995, 55, 89-108)

L'avantage des organismes transgéniques de l'invention réside dans le fait qu'ils expriment de manière constitutive le peptide anti-microbien, de l'invention.

L'invention a également pour objet un anticorps, polyclonal ou monoclonal, dirigé contre au moins l'un des peptides selon l'invention.

Toute méthode de préparation des anticorps connue de l'art antérieur peut être mise en oeuvre pour obtenir les anticorps de l'invention.

L'invention a en outre pour objet un procédé de détection du polypeptide selon l'invention, caractérisé en ce que dans une première étape on met en contact un milieu susceptible de contenir ledit peptide et un anticorps selon l'invention et dans une deuxième étape on détecte les complexes formés par l'anticorps et le peptide. Ce procédé est particulièrement intéressant pour détecter des traces de biopesticides dans un environnement particulier.

L'invention a également pour objet un procédé de traitement anti-microbien, et/ou cytotoxique dans lequel on met en contact par tout moyen approprié (application, épandage, pulvérisation, ...) un organisme, particulièrement un végétal, et au moins un agent anti-microbien et/ou cytotoxique choisi parmi au moins l'un des peptides tels que décrits précédemment et/ou l'un des vecteurs et/ou l'un des polynucléotides et/ou l'un des systèmes biologiques et/ou une composition, précédemment décrits.

En agriculture, le procédé de traitement de l'invention peut être mis en application selon toutes les formes utilisées en agriculture, comme par exemple de façon traditionnelle en application externe en champ, par exemple par pulvérisation ou épandage d'une composition liquide de l'invention, en additif d'enrobage de graines ou dans une stratégie de plantes transgéniques.

Outre les dispositions qui précèdent, l'invention comprend encore d'autres dispositions qui ressortiront de la description qui va suivre, qui se réfère à des exemples de mise en oeuvre de l'invention ainsi qu'aux dessins annexés, dans lesquels :
- La figure 1 présente les résultats obtenus lors d'une amplification PCR sur les ADN complémentaires obtenus à partir d'ARN de jeunes siliques *d'Arabidopsis thaliana.*
- La figure 2 présente les résultats des essais réalisés en vue de mettre en évidence le (ou les) codons d'initiation (ATG) fonctionnels dans le transcrit SUP25. Dans le panneau 2A, la flèche indique la position des ATG et l'octogone la position des codons stop.
- La figure 3 présente les résultats d'un test de phytotoxicité du peptide (SUP16) de 16 acides aminés consistant en la séquence SEQ ID NO : 1 et du peptide (SUP25) de 25 acides aminés consistant en la séquence SEQ ID NO : 10.
- La figure 4 présente les résultats des essais réalisés en vue de déterminer la concentration optimale pour l'activité cytotoxique du peptide (SUP25) de 25 acides aminés consistant en la séquence SEQ ID NO : 10.
- Les figures 5A et 5B présentent les résultats d'une expérience de marquage TUNEL sur des protoplastes soumis à différents variants du peptide.

Les exemples suivants sont illustratifs de l'invention et ne la limitent aucunement.

### EXEMPLE 1 : Matériel et Méthodes

### 1) Nomenclature :

SUP25 et pep25 désignent le peptide de 25 acides aminés correspondant à la séquence SEQ ID NO : 10.

SUP16 et pep16 désignent le peptide de 16 acides aminés correspondant à la séquence SEQ ID NO : 1.

PepDS désigne un peptide SUP25 dont les acides aminés 4 à 15 ont été retirés.

Pep1S désigne un peptide SUP 25 dont la cystéine en position 23 a été remplacée par une sérine.

Pep2S désigne un peptide SUP 25 dont les cystéines en position 16 et 23 ont été remplacées chacune par une sérine.

### 2) Matériel végétal

Toutes les expériences ont été réalisées sur *Arabidopsis thaliana.* Deux écotypes sauvages ont été utilisés :
➢ Columbia (Col-0) pour l'ensemble des transformations par la méthode *in planta.*
➢ C24, écotype ayant servi à la confection du piège à promoteur.

### 3) Matériel bactérien

*Escherichia coli :* souche DH5ct
*Xanthomonas campestris* pv campestris : souche 8004
*Pseudomonas syringae* pv tomato : souche DC3000
*Erwinia amylovora :* souche 1430

### 4) Matériel nucléique

### 4. 1. Plasmides

### 4.1.a. Plasmides de clonage de fragments PCR et de produits de digestion.

Les plasmides qui ont été utilisés pour cloner les fragments PCR en utilisant le principe du "TA cloning" sont le pMOSBlue®, vendu par la société Amersham, les vecteurs pGEM®-T et pGEM®-T easy vendus par la société Promega. Ces trois vecteurs contiennent le gène conférant la résistance à l'ampicilline aux bactéries qui les contiennent. De plus, ils portent le gène *lacZ* qui permet une sélection des plasmides ayant effectivement incorporé un insert. Leur hôte est la bactérie *E. coli* (DH5α) Ces mêmes plasmides, refermés sur eux-mêmes, ou encore le vecteur pBluescript II SK® (pBSK, Stratagène), ont été utilisés pour le clonage de produits de digestion.

### 4.1.b. Plasmides de clonage utilisés comme vecteur de transformation transitoire :

Tous les plasmides cités ci-dessus peuvent servir de vecteur d'expression transitoire.
➢ Pour les fusions traductionnelles le plasmide utilisé a été pRTL2-EGFP (Restrepo A, Freed DD, Carrington JC., Nuclear transport of plant potyviral proteins. Plant Cell. 1990 Oct. ; 2 (10) : 987-98).
➢ Pour la construction des cassettes d'expression sens et antisens de la protéine SUP25, l'amplification PCR du gène ou de l'antisens est faite grâce aux amorces pepS-3X et pepS-5H ou pepAS-5S et PepAS-3H. Le fragment amplifié est cloné dans pGEM^{®}-T, l'insert est excisé par les enzymes de restriction *Nco*I et *Not*I puis inséré dans pRTL2-EGFP linéarisé par les mêmes enzymes et purifié sur gel d'agarose.

### 4.2. Oligonucléotides

Les séquences des amorces utilisées sont données ci-dessous.

| N° | Nom | Séquence | Objet |
|---|---|---|---|
| SEQ ID NO : 21 | PepS-5H | CAAGTAAGCTT GCTCAGTTG | Clonage de l'ORF SUP25 en fusion avec les opérateurs dans le vecteur binaire |
| SEQ ID NO : 22 | PepS-3X | GCTCTAGATAC TTAGAGATGAC AGAGACG | Clonage de l'ORF SUP25 en fusion avec les opérateurs dans le vecteur binaire |
| SEQ ID NO : 23 | PepAS-5S | ACTGAGCTCGT TGTATTTTTAAT CGAATGG | Clonage de l'anti SUP25 en fusion avec les opérateurs dans le vecteur binaire |
| SEQ ID NO : 24 | PepAS-3H | ACGGAAGCTTT ACTTAGAGATG ACAGAGACG | Clonage de l'anti SUP25 en fusion avec les opérateurs dans le vecteur binaire |
| SEQ ID NO : 25 | OTi-05 | CGTCTTCGAGA AAAGTGTTAG | RT-PCR |
| SEQ ID NO : 26 | OEx-Ti-15 | ATCAGTCAGAC AGTCAAATTC | RT-PCR |
| SEQ ID NO : 27 | OPend3 | TACTTAGAGAT GACAGAGACG | RT-PCR |

D'autres amorces commerciales comme T7, T3, T7 term, SP6 et U19 ont été utilisées en routine dans les expériences de clonage.

### 5) Milieux de culture et solutions

### 5. 1. Milieu de culture pour plantes

Milieu de germination MS : milieu Murashige et Skoog avec vitamine B5 (Duchefa) 2,2 g/l ; MES (Sigma) 0,5 g/l ; glucose 5g/l ; pH 5,7 avec KOH 1 M ; Plant agar 7g/l (Duchefa).

### 5.2. Milieux de sélection :

Antibiotiques :
Kanamycine : 50 mg/l. (sélectionne l'activité néomycine phosphotransférase du gène *nptII*).
Hygromycine : 30 mg/l. (sélectionne l'activité hygromycine phosphotransférase du gène *hpt*).
Méthotréxate : 0,1 mg/l (sélectionne l'activité dihydrofolate réductase du gène *dhfr*).

### 5.3. Milieux de culture pour Escherichia coli :

Milieu LB (Luria-Bertani) liquide : extrait de Levure 5g/l ; bactotryptone 10g/l ; NaCl 10g/l.

Milieu 2XL liquide : extrait de Levure 10g/l ; bactotryptone 20g/l ; NaCl 1g/l ; glucose 2 g/l.

Milieu Terrific Broth liquide : extrait de Levure 24 g/l ; bactotryptone 12 g/l ; glycérol 4 ml/l ; KH₂PO₄ 4,62g/l ; K₂HPO₄ 25,08g/l.

Milieux solides : comme milieux liquides + 15 g/l Bacto agar (Difco).

Antibiotiques pour *E. coli :* ampicilline : 100 mg/l ; kanamycine : 50 mg/l ; chloramphénicol (stock dans l'éthanol) : 12,5 mg/l.

### 5.4. Solutions et tampons :

### 5.4.a. Tampons relatifs aux extractions d'acides nucléiques :

Tampon d'extraction de l'ADN génomique de plante :
Méthode Dellaporta : Tris-HCl (pH 8) 100 mM ; EDTA (pH 8) 50 mM ; NaCl 0,5M ; β-mercaptoéthanol 10 mM.
Tampon d'extraction de l'ARN de plante (REB) : Tris-HCl (pH 8) 25 mM ; EDTA 25 mM ; NaCl 75 mM ; SDS 1%.
Tampon TE : Tris-HCl (pH 8) 10 mM ; EDTA (pH 8) 2 mM.

### 5.4.b. Tampons relatifs aux électrophorèses en gels d'agarose :

➢ Gels d'ADN
   Tampon TBE : Tris-HCl 89 mM ; acide borique 89 mM ; EDTA (pH 8) 2 mM.
   Tampon TAE : Tris-HCl 40 mM ; acide acétique 40 mM ; EDTA (pH 8) 1 mM.
   Tampon de charge ADN 10x : xylène cyanol FF 0,25% ; bleu de bromophénol 0,25% ; glycérol 30%.
➢ Gels d'ARN
   Tampon MOPS 5x : MOPS 0,1 M ; acétate de sodium 40 mM; EDTA (pH 8) 5 mM.
   Tampon de charge ARN, par échantillon : tampon MOPS 5x 2 µl; formaldéhyde 3,5 µl ; formamide 10 µl, bromure d'éthidium (BEt) à 1 mg/ml 0,2 µl.

### 5.4.c. Tampons relatifs aux hybridations de type Southern blot

Tampon de dépurination : HCl 0,25 N.
Tampon de dénaturation : NaOH 0,5 N - NaCl 1,5 M.
SSC 20 x : NaCl 3 M ; citrate de sodium 0,3 M.
SSPE 20 x : NaCl 3,6 M ; NaH₂PO₄ 0,2 M ; EDTA (pH 7,7) 0,02 M.
Denhardt's 50 x : Ficoll^{™} 10 g/l ; PVP (polyvinylpyrrolidone) 10 g/l ; BSA 10 g/l.
Tampon d'hybridation : EDTA 10 mM ; SSPE 6x ; SDS 0,5% ; Denhardt's 5x.
Tampon de lavage I : SSC 2x ; SDS 0,1 %.
Tampon de lavage II : SSC 1x ; SDS 0,1%.

### 5.4.d. Tampons relatifs à l'étude de l'activité enzymatique GUS

### ➢ Histochimie

Tampon de coloration GUS : KH₂PO₄ 40 mM ; KHPO₄ 60 mM, 5-bromo-4-chloro-3-indoylglucuronide (X-gluc, Biosynth AG, Switzerland) en solution dans le diméthyl formamide (DMF) 1 mg/ml ; azide de sodium 0,02% ; NaCl 50 mM ; ferricyanure de sodium 0,5 mM ; ferrocyanate de sodium 0,5 mM ; Triton X 100 0,1% (v/v).

### ➢ Spectrophotométrie

Tampon d'extraction GUS ou GFP : Tris-HCl (pH 7) 50 mM ; SDS 0,1% ; EDTA 10 mM ; dithiotréitol (DTT) 3 mM.

### 5.4.e. Tampons pour les immunoempreintes de protéines

Tampon d'extraction pour immunoempreintes : Tris-HCl (pH 8,3) 10 mM ; NaCl 10 mM.

Tampon de charge en gel d'acrylamide : Tris-HCl (pH 6,8) 125 mM ; SDS 4% ; glycérol 10% ; DTT 0,2 M ; bleu de bromophénol 0,2%.

Colorant au Bleu de Coomassie : Bleu de Coomassie R250 0,5 g/200 ml ; éthanol 90% ; acide acétique 10%.

Solution décolorante : éthanol 10% ; acide acétique 10% ; glycérol 3,5%.

Tampon de transfert : Tris-base 20 mM ; glycine 150 mM ; méthanol 20% (v/v)

TBS : Tris-base 2,42 g/l ; NaCl 8 g/l ; ajusté à pH 7,6.

TRS-T : TBS + 0, 1 %, Tween 20.

Lait écrémé.

### 6) Méthodes :

### 6.1) Méthodes de physiologie végétale

### 6. 1.1) Conditions de culture

### ➢ Culture en terre

Les cultures en terre ont été réalisées dans un mélange de terreau horticole et de vermiculite, stérilisé par un autoclavage à 125 °C pendant 20 minutes. Les plantes ont été cultivées dans une chambre de culture, à 20-22 °C, en lumière continue.

### ➢ Culture in vitro

Pour la culture *in vitro,* les graines ont été stérilisées sous hotte à flux laminaire, par incubation dans un produit stérilisant (Domestos, Unilever) à 10% (v/v) dans de l'eau distillée stérile, pendant 10 minutes. Puis elles sont rincées 5 fois dans de l'eau distillée stérile. Les graines sont ensuite mises à germer dans des boîtes de Pétri (20 x 100 mm) contenant du milieu gélosé MS, avec ou sans antibiotique. Les plantes ont été cultivées dans une chambre de culture, à 20-22 °C, en jours continus.

### 6.2) Méthodes de biologie moléculaire

### 6. 2.1) Manipulation de l'ADN

### 6.2. 1. A) Méthodes d'extraction

### ➢ ADN plasmidique

L'ADN bactérien plasmidique a été extrait selon une procédure de lyse alcaline et purifié à l'aide de deux kits différents selon la quantité de plasmide requise : le kit "High Pure Plasmid isolation Kit" de Boehringer Mannheim a été utilisé pour réaliser des mini-préparations de plasmides (environ 20 µg) à partir de 5 ml de culture saturée (*E. coli* souche DH5α). Ce kit utilise une colonne de silice pour retenir l'ADN. Le kit "Wizard™ Plus Midiprep DNA Purification System" de Promega a été utilisé pour extraire de plus grandes quantités de plasmide (environ 100 µg pour les plasmides à grand nombre de copies) à partir de 50 ml de culture saturée (*E. coli* souche DH5α). Ce kit utilise une colonne d'hydrochlorure de guanidine pour retenir l'ADN.

### ➢ ADN génomique

Méthode Dellaporta : cette méthode a été employée pour extraire rapidement et facilement de nombreux lots d'ADN génomique destinés à la vérification PCR des plantes transgéniques. Ce protocole, décrit par Dellaporta (Dellaporta et al., PMB, 1983, 1, 19-21), a été adapté à de plus petits volumes pour être réalisable en tubes de 500 µl. Chaque extrait a été obtenu à partir de 2 à 4 feuilles (selon leur taille) d'une même plante *d'Arabidopsis thaliana.*

### 6.2.1.b) Réaction de Polymérisation en Chaîne (PCR) :

### ➢ Sur l'ADN plasmidique ou génomique, après extraction :

Les réactions sont réalisées dans des tubes de 500 µl, dans un volume final de 25 µl :
"mix" : mélange des constituants suivants, préparé pour toutes les réactions PCR :
   10 pmoles de chacun des deux oligonucléotides spécifiques,
   11 µM de chaque dNTP,
   2,5 µl d'un tampon 10x, fourni par la compagnie commercialisant la polymérase,
   1,25 mM de MgCl₂ (ou MgSO₄, selon l'enzyme utilisée),
   1 unité d'ADN-polymérase thermostable,
   ADN matrice (fonction de la polymérase employée et du type d'ADN matrice),
   qsp 25 µl d'eau dessalée, déionisée et stérile.

Pour les réactions PCR classiques ne nécessitant pas l'obtention d'un produit de séquence strictement identique à celle de la matrice, les polymérases d'ADN thermostables Dynazyme (Ozyme), Taq (Promega) et TfI (Promega) ont été utilisées.

Dans ce cas, 5 ng d'ADN plasmidique ou 200 ng d'ADN génomique suffisent à la réalisation de l'expérience.

Pour obtenir un fragment amplifié dont la séquence est identique à celle de la matrice, l'enzyme de fidélité Pfu (Stratagène) a été utilisée. Cette enzyme requiert une quantité de matrice plus importante : classiquement 100 à 150 ng de plasmide ont été utilisés, selon les recommandations du fournisseur. Dans le cas d'une matrice d'ADN génomique, 500 à 1000 ng ont été utilisés.

Enfin, lorsqu'un produit PCR de taille supérieure à 2 kb était attendu, la PfuTurbo™ (Stratagène) a été choisie. Les quantités d'ADN matrice utilisées sont alors de 10 ng pour l'ADN plasmidique et environ 500 ng pour l'ADN génomique.

Les conditions de réaction sont :
- Dénaturation: 93°C, 3 min
- Dénaturation: 93°C, 1 min
- Hybridation: entre 48 et 55°C, 1 min
- Elongation: 72°C, variable**
- Extension finale: 72°C, 10 min
**variable selon le couple d'amorces
Tfl et PfuTurbo™ : 1 min/kb à amplifier
Pfu : 2 à 3 min/kb à amplifier

Le nombre n de cycles PCR est compris entre 30 et 40 pour les réactions mettant en jeu la Dynazyme (Ozyme), la Taq (Promega) et la Tfl (Promega). Pour les réactions réalisées avec les enzymes de fidélité, le nombre de cycles est plus faible (jusqu'à 25), de façon à limiter les erreurs.

### ➢ Sur l'ADN plasmidique contenu dans des cellules bactériennes :

L'amplification d'une séquence plasmidique peut directement se faire à partir de colonies bactériennes, ceci pour rechercher les colonies correspondant aux bactéries recombinantes (ayant effectivement intégré le plasmide contenant l'insert d'intérêt) Le "mix" PCR (même composition que celle donnée ci-dessus) est préparé et réparti par 25 µl dans plusieurs tubes de 500 µl. L'enzyme utilisée est la Dynazyme (Ozyme), la Taq (Promega) ou la Tfl (Promega).

Les colonies sont prélevées à la surface du milieu de culture solide à l'aide de tout outil de prélèvement approprié (cône, loupe, etc.), repiquées sur une boite neuve contenant du milieu de culture LB solide, et l'outil de prélèvement est plongé dans le "mix" et brièvement agité.

Les conditions PCR sont les mêmes que celles données précédemment.

Dans tous les cas, 10 µl de produit PCR auxquels est ajouté 1 µl de tampon de charge ADN 10x, sont déposés dans un gel d'agarose (pourcentage d'agarose variable en fonction de la taille du fragment amplifié à observer, tampon TBE).

### 6.2.1.c) Méthodes de clonage :

Le clonage moléculaire passe par différentes étapes dont l'isolement du fragment à cloner (après action d'une endonucléase de restriction ou non - cas de produits PCR à cloner directement dans un vecteur de type "TA cloning" -), l'ouverture du vecteur de clonage (sauf dans le cas des vecteurs de type "TA cloning"), la ligation de ces deux éléments et l'amplification de la nouvelle construction après son introduction dans une cellule bactérienne.

### ➢ Création du plasmide recombinant :

- Hydrolyse par des enzymes de restriction ou digestion :
   Les enzymes de restriction sont utilisées dans les conditions préconisées par les fournisseurs. Le volume d'enzyme utilisé correspond au maximum à 10% du volume final de la réaction de digestion.
- Déphosphorylation des extrémités après digestion :
   La stratégie de déphosphorylation a été utilisée lorsqu'il était nécessaire d'empêcher un vecteur digéré par une seule enzyme de restriction de se refermer sur lui-même. Dans ce cas, la phosphatase alcaline de Boehringer Mannheim a été employée selon les recommandations du fournisseur.
- Purification des fragments d'ADN
   Les purifications des fragments d'ADN ont été réalisées de 3 façons différentes selon le cas :

### Dialyse

Lorsqu'il s'agissait uniquement de dessaler l'ADN (après action d'une endonucléase de restriction par exemple), selon le cas et le volume à traiter, une simple membrane Millipore, une "spin column" (Sephadex G-50) ou encore une précipitation à l'éthanol suivie d'un lavage du culot obtenu à l'éthanol 70% ont été réalisés.

### Phénol/chloroforme

Lorsqu'en plus des sels, une élimination des protéines (enzyme de restriction ou phosphatase alcaline) s'avérait nécessaire, une purification par le mélange phénol / chloroforme suivie d'une précipitation à l'éthanol a été réalisée.

### A partir d'un gel d'agarose

De façon à séparer le fragment d'ADN d'intérêt d'autres fragments d'ADN contaminants, une électrophorèse en gel d'agarose (tampon TAE) a été réalisée. Le fragment d'intérêt a ensuite été extrait du gel grâce au kit "QIAQuick Gel Extraction Kit" de Qiagen ou "Wizard™ PCR Prep" de Promega : le morceau d'agarose contenant l'ADN est rendu liquide par chauffage puis passé sur une colonne qui fixe l'ADN. Après une série de lavages, l'ADN est élué avec de l'eau et peut ainsi être utilisé directement pour le clonage dans un vecteur.
- Ligation de l'insert et du vecteur :
   Les ligations entre les fragments d'ADN à intégrer et les vecteurs de clonage aux sites compatibles sont effectuées en présence d'une unité d'ADN ligase du phage T4 (Promega ou Boehringer Mannheim) préférentiellement à 16°C pendant une nuit ou bien de 2 à 4 heures à température ambiante. La quantité totale d'ADN utilisée est comprise entre 200 ng et 500 ng, en respectant un rapport molaire entre le vecteur et l'insert de 1 : 3 à 1 : 5, dans un volume final de 10 à 20 µl, correspondant aux conditions préconisées par le fournisseur.
- Ligation d'un adaptateur
   Les oligonucléotides sont choisis pour s'apparier en laissant les extrémités cohésives désirées. Les oligonucléotides sont hybridés à 1 pmol/µl dans 10 µl de tampon tfl 1X. Ils sont incubés 1 min à 95°C puis 10 min à 45°C. Ensuite 20 ng d'oligonucléotides (dans un volume maximal de 2 µl) et 100 ng de vecteur sont introduits dans la réaction de ligation.

### ➢ Cas particulier du clonage direct d'un produit PCR

Dans le cas particulier du clonage direct d'un produit obtenu par PCR, on utilise les enzymes Dynazyme (Ozyme), Taq (Promega) ou Tfl (Promega) qui ont la particularité de rajouter de temps en temps une adénosine aux extrémités 3' de l'ADN double brin. Des systèmes de ligation exploitant cette particularité ont été développés : il s'agit des systèmes pMOSBlue^{®} (Amersham), pGEM^{®}-T et pGEM^{®}-T easy (Promega), mettant en oeuvre un vecteur digéré par l'enzyme *Eco*RV*,* générant des bords francs et ajout d'une thymidine aux extrémités 3' des vecteurs. La liaison entre le produit PCR, purifié à partir d'un gel d'agarose, et le vecteur utilise ainsi le principe dit du "TA cloning". La réaction de ligation est réalisée dans 10 µl, avec la ligase fournie dans le kit et selon les instructions du fabricant.

Lorsque l'utilisation des enzymes de fidélité Pfu ou PfuTurbo™ (Stratagene) - qui ne rajoutent pas d'adénosine aux extrémités 3' de l'ADN amplifié - était requise, la technique de "A-tailing" a été mise en oeuvre : elle consiste à prélever 3 µl du produit PCR obtenu, à y rajouter 1 µl de tampon 10x, 1 µl de MgS0₄ à 25 mM, 1 µl de dATP à 2mM, 1 µl de Tfl (à 5 unités / µl) et 3 µl d'eau désallée, déionisée et stérile. L'ensemble est incubé 30 min à 70°C puis dialysé 1 heure à température ambiante contre de l'eau désallée, déionisée et stérile, Le fragment ainsi préparé est prêt à un emploi direct pour la réaction de ligation dans un vecteur « TA cloning ».

### ➢ Préparation et transformation de bactéries compétentes

Les plasmides sont intégrés par choc thermique suivant le protocole décrit par Sambrook et al. (Molecular cloning : a laboratory manuel, 2nd Ed., 1989).

Les bactéries recombinantes sont alors détectées par amplification PCR au moyen d'amorces présentes dans le vecteur et/ou l'insert, selon le protocole décrit ci-dessus.

### 6.2.1.d) Séquençage :

Les réactions de séquence ont été réalisées par la méthode de terminaison d'extension de chaîne (Sanger et al., PNAS, 74, 5463-5467, 1977) en présence de didéoxynucléotides, chacun marqué par un fluorochrome différent (PRISM Read Reaction DyeDeoxy Terminator Cycle Sequence Kit, Applied Biosystems). Chaque réaction nécessite 250 ng d'ADN matrice double brin et 5 pmoles d'amorce dans un volume final de 10 µl contenant 4 µl du mélange Dye deoxy Terminator dilué 4 fois. Les produits de réaction ont été analysés par électrophorèse en utilisant un séquenceur AB1377A.

### 6.2.1.e) Hybridation de type Southern blot

### ➢ Préparation des sondes radioactives

Les sondes utilisées dans les expériences d'hybridation de type Southern blot ont été marquées au phosphore 32 (³²P) par la technique de PCR : elle consiste à incorporer de l'α³²P-dCTP (3000 Ci/mmole). Le marquage par PCR est réalisé de la façon suivante : le mélange réactionnel, d'un volume final de 25 µl, contient 2 ng de plasmide contenant la sonde, 2 pmoles de chaque amorce spécifique, 50 µCi d'α³²P-dCTP, 3,6 µM de dCTP froid, 60 µM des autres dNTP froids et 1 µl de Tfl (à 5 unités / µl). Les réactions de PCR sont réalisées comme précédemment décrit et comprennent 20 à 25 cycles.

La qualité des sondes obtenues est testée sur gel d'agarose transféré sur membrane de nylon et exposé 2 minutes sur un film radiographique.

La sonde radioactive est alors purifiée sur colonne à l'aide du système MicroSpin7m G25 Columns (Amersham) avant d'être utilisée.

### 6.2.2) Manipulation de l'ARN :

### 6.2.2.a) Méthode d'extraction

Les ARN totaux ont été extraits à partir de différents tissus *d'Arabidopsis thaliana* à l'aide du tampon d'extraction REB et selon le protocole de Kay et al. (Science, 236, 1299-1302, 1987). Les ARN polyA+ sont obtenus à l'aide d'un kit polyAtract (Promega) selon les indications du fournisseur.

### 6.2.2.b) Hybridation d'ARN : Northern blot

### ➢ Préparation des sondes radioactives

Les sondes utilisées dans les expériences d'hybridation de type Northern blot ont été marquées au phosphore 32 par la technique de PCR comme décrit précédemment.

### ➢ Gel, transfert sur membrane et hybridation

Jusqu'à 50 µg d'ARN totaux, dans un volume de 4,5 µl, ont été ajoutés à 15,5 µl de tampon de charge, puis dénaturés pendant 5 min à 65 °C. Ils ont été déposés dans un gel d'agarose à 1% (tampon MOPS 1x, formaldéhyde 2,2 M) et séparés par électrophorèse dans du tampon MOPS 1x (5 min à 125V, puis 2h30 à 75V). Les ARN ont été colorés au bromure d'éthidium, afin de s'assurer d'une charge homogène entre les différentes pistes. Après deux passages de 15 min du gel dans le tampon de lavage, les ARN ont été transférés pendant une nuit sur des membranes de nylon (membranes Hybond N+, Amersham) par capillarité (Sambrook et al., Molecular cloning : a laboratory manuel, 2^{nd} Ed., 1989) en présence d'une solution de SSC 20x. Les ARN ont été fixés sur les membranes par une incubation de 2 heures à 80°C. La préhybridation a été réalisée dans 25 ml ou 50 ml (suivant la taille des membranes) de tampon d'hybridation contenant 20 mg/l d'ADN de thymus de veau dénaturé. Après 30 min à 2 h de préhybridation à 65°C, la sonde ADN dénaturée est ajoutée dans le tampon pour une incubation de 16 h à 65°C. Les membranes sont ensuite lavées 2 fois 20 min à 65°C avec le tampon de lavage I. Puis un dernier lavage est réalisé à 65°C pendant 10 min maximum avec le tampon II. Les signaux d'hybridations ont été détectés soit par exposition de film BIOMAXTm (Kodak) soit par analyse d'image au Phosphorimager (Storm 640, Molecular Dynamics). Pour des hybridations successives, les membranes ont été déshybridées selon les recommandations données dans le manuel Amersham.

### 6.2.2.c) Méthode de RT-PCR

L'approche de PCR par transcription inverse a été utilisée dans ce travail, pour chercher, dans les jeunes siliques, des transcrits correspondant à la région d'ADN étiqueté. La méthode se divise en 2 étapes successives : l'obtention d'ADN complémentaires (ADNc) à partir d'ARN totaux, puis la réaction PCR proprement dite, à partir de ces ADNc.

### ➢ Obtention d'ADNc

La réaction de transcription inverse a été réalisée à l'aide du kit ProSTARTulirst-Strand RT-PCR Kit (Stratagene), à partir d'ARN totaux traités à la DNase 1 (de façon à limiter les contaminations des futurs ADNc par de l'ADN génomique. Le kit utilise la transcriptase inverse du virus de la leucémie murine de Moloney (MuMLV) et une amorce oligodT.

### ➢ PCR sur les ADNc

Les produits de la transcription inverse sont utilisés comme matrice pour la PCR. Les conditions sont les mêmes que celles décrites précédemment (cf. le paragraphe concernant la PCR) La seule différence réside dans le volume final de réaction (50 µl) et le nombre de cycles, plutôt élevé (30 à 45 cycles selon le cas).

### EXEMPLE 2 : Mise en évidence d'un transcrit SUP25 :

### 1) Mise en évidence du messager correspondant à la protéine SUP25 :

### 1.1) Extraction des ARN d'Arabidopsis thaliana :

Les ARN totaux ont été extraits à partir de différents tissus *d'Arabidopsis thaliana* à l'aide du tampon d'extraction REB et selon le protocole de Kay et al. (Science, 1987, 239 : 1299-1302). Les ARN polyA+ sont obtenus en utilisant le kit polyAtract (Promega), selon les instructions du fournisseur.

### 1.2) Hybridation de type Northern :

Les techniques classiques d'hybridation de type Northern n'ont jamais permis de mettre en évidence la présence du messager correspondant à la protéine SUP25 dans des extraits de siliques immatures *d'Arabidopsis thaliana* (Varoquaux, thèse, Université de Perpignan, France, 2000).

Le niveau d'expression du gène natif correspondant apparaît donc en dessous du seuil de détection de la technique.

Afin d'augmenter la sensibilité de l'expérience, l'expression du gène a été analysée par la technique de Northern sur gel de polyacrylamide avec des extraits d'ARN polyA⁺. Aucun signal d'hybridation n'a pu être observé sur des extraits de 4 µg témoignant de l'absence ou de la très faible expression du gène.

1.3) Réaction de polymérisation en chaîne par transcription inverse (RT-PCR) :

Afin de rechercher un signal dans les jeunes siliques *d'Arabidopsis thaliana.,* la technique de la transcription inverse RT-PCR est utilisée.

L'expérience est réalisée en 2 étapes : une transcription inverse des ARN totaux de siliques est réalisée, suivie d'une PCR sur les ADNc polyA+ ainsi obtenus.

### 1.3.a) Obtention d'ADN complémentaires (ADNc) :

La réaction de transcription inverse a été réalisée à l'aide du kit ProSTARTulirst-Strand RT-PCR Kit (Stratagene), à partir de 10 µg d'ARN totaux traités à la DNase 1 (de façon à limiter les contaminations des futurs ADNc par de l'ADN génomique). Le kit utilise la transcriptase inverse du virus de la leucémie murine de Moloney (MuMLV) et une amorce oligodT.

### 1.3.b) PCR sur les ADNc :

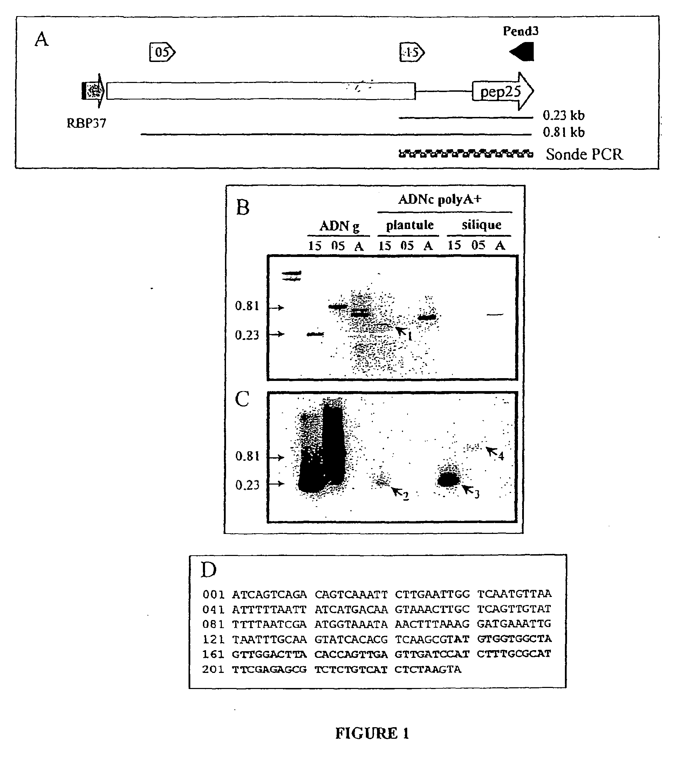

Les 2 couples d'amorces choisis (05, Pend3) et (15, Pend3) sont présentés dans la figure 1A.

L'amplification sur l'ADN génomique (figure 1B, pistes ADNg 05 et 15) permet de vérifier la taille des produits de réaction et d'apprécier la qualité d'amplification. Les produits d'amplification réalisés à l'aide de ces 2 couples doivent permettre de mettre en évidence 2 fragments respectivement de 0,23 kb (couple 15, Pend3) et de 0,81 kb (couple 05, Pend3).

L'amplification d'un gène d'actine 2 ubiquitaire (pistes A), permet de contrôler le niveau d'amplification des ARN et d'évaluer la contamination d'ADN génomique par la présence d'un intron entre les 2 amorces utilisées.

2,5 µl de produit de la transcription inverse (α) sont utilisés comme matrice pour la PCR. Les conditions sont les mêmes que celles décrites précédemment (cf. le paragraphe concernant la PCR). La seule différence réside dans le volume final de réaction (50 µl) et le nombre de cycles, plutôt élevé (30 à 45 cycles selon le cas).

Les produits obtenus de la réaction PCR sont ensuite soumis à une hybridation selon Southern après électrophorèse en gel d'agarose, avec une sonde PCR radioactive correspondant à la région du peptide (figure 1A) Les résultats de cette hybridation sont présentés à la figure 1C.

Du transcrit d'actine montre que le niveau d'ARN est très inférieur dans l'extrait de siliques par rapport à l'extrait de plantules (figure 1B).

La flèche 3 (figure 1C) révèle la présence du produit d'amplification à la taille prédite de 0,23kb attestant de l'existence du transcrit dans des extraits d'ARN polyA+ de siliques immatures ; la flèche 4 montre un signal négligeable à la taille attendue de 0,81kb. La contamination de l'extrait par l'ADN génomique est donc très faible.

Le transcrit de 0,23kb est présent dans les extraits de siliques immatures (0 à 5 jours après fécondation), ainsi que dans les plantules à un niveau plus faible (figure 1C, flèche 2), si on compare à l'actine.

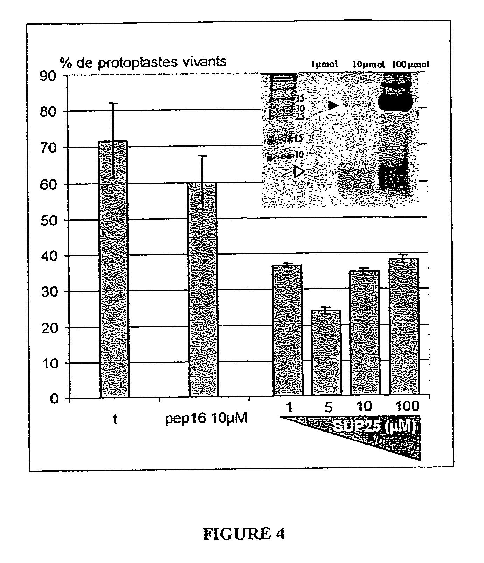

On note la présence d'une bande artefactuelle dans la figure 4B (flèche 1) ; ce produit d'amplification a été cloné puis séquencé et ne contient aucune homologie avec le gène de l'invention, cette bande est d'ailleurs absente du profil d'hybridation (figure 1 C).

Le séquençage du produit de 0,23kb (transcrit SUP25) a révélé une séquence de 227 paires de bases présentée à la figure 1D.

### 2.) Mise en évidence d'ATG fonctionnels dans le transcrit :

L'analyse de la séquence amplifiée par RT-PCR du transcrit SUP25 montre l'existence de codons d'initiation (ATG) donc de trois phases ouvertes de lecture (ORF).

La séquence en acides aminés déduite de la plus grande phase ouverte de lecture correspond à un peptide de 25 acides aminés (SUP 25).

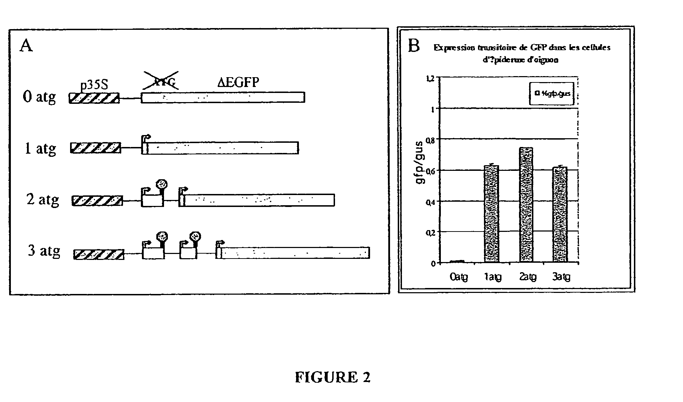

Pour démontrer l'aptitude de la machinerie traductionnelle à initier la synthèse protéique sur les ATG, une expérience d'expression transitoire a été menée. La "Green Fluorescent Protein" (GFP) (plasmide PRTL2-EGFP) dépourvue de son codon d'initiation est clonée en fusion traductionnelle avec les différents ATG (figure 2A, 0 atg : pas d'ATG, 1 atg : 1 ATG, et ainsi de suite). Chaque construction ainsi clonée est co-transfectée dans l'épiderme d'oignon avec le plasmide pRTL2-GUS (Restrepo A, Freed DD., Carrington JC., Nuclear transport of plant potyviral proteins. Plant Cell. 1990 Oct. ; 2 (10) : 987-98) en rapport molaire constant, permettant ainsi de disposer d'un contrôle interne. Sur chaque échantillon, la sous-population de cellules exprimant la GFP est dénombrée puis rapportée à la sous-population exprimant la protéine GUS, chaque rapport de spots ainsi évalué reflète le niveau d'expression du gène rapporteur (figure 2B). Une première construction sans ATG permet de vérifier, dans un contrôle négatif, que la GFP sans ATG n'est pas traduite, aucune cellule exprimant la GFP n'a été comptée. Pour les trois autres fusions traductionnelles, le niveau d'expression de la GFP est sensiblement le même, attestant ainsi du caractère fonctionnel des 3 ATG. La présence des phases de lecture de petite taille en amont de la séquence codante n'affecte pas le niveau d'expression de la GFP. Ceci indique que les codons stop des phases de lecture de petite taille n'imposent pas l'arrêt du complexe ribosomique qui est alors capable de ré-initier la traduction en aval sur le transcrit. En d'autres termes, le résultat de cette expérience démontre sans ambiguïté que la traduction peut être initiée ou ré-initiée sur le troisième ATG du locus. Ce résultat permet ainsi d'étayer l'hypothèse concernant l'existence du peptide.

### EXEMPLE 3 : Immunoempreintes :

### 3.1) Préparation d'anticorps dirigés contre le peptide :

Les peptides ont été synthétisés par la société Eurogentec. Les anticorps ont été produits sur le peptide synthétique tronqué (sup 16) par la société Eurogentec.

### 3.2) Extraction de protéines de siliques :

Les protéines ont été extraites par broyage des siliques dans le tampon d'extraction pour immunoempreintes, à l'aide de mini-pilons (Kontes) reliés à un broyeur RZE2021 (Heidolph). Après centrifugation (20 minutes à 10000 g), la concentration protéique du surnageant a été déterminée par la méthode de Bradford à l'aide du réactif vendu par la société Bio-Rad.

### 3.3) Analyses électrophorétiques :

Les électrophorèses en gel de polyacrylamide (SDS-PAGE) ont été réalisées selon la méthode décrite par Laemmli (1970). Le gel de concentration est à 3% d'un mélange acrylamide / bis acrylamide (30 % / 0,8 %) et le gel de séparation est à 18% du mélange Prosieve. 15 µg de protéines totales auxquelles on ajoute le tampon de charge à la concentration finale de 1 x, sont portés à 100°C pendant 3 minutes puis déposés dans chaque piste.

Les protéines sont séparées par un courant constant de 15 à 20 mA par gel, pendant environ 1 heure. Les gels qui ne sont pas destinés aux immunoempreintes sont colorés au Bleu de Coomassie pendant 30 minutes puis décolorés jusqu'à apparition des bandes correspondant aux protéines.

### 3.4) Immunoempreintes :

Pour les immunoempreintes, les protéines ont été transférées après migration sur gel, sur des membranes de nitrocellulose (Pure Nitrocellulose membrane, 0,1 µm, Protan) dans une cuve d'électrotransfert semi-sec Temblot Ae-6675 (Touzart et Matignon) pendant 40 minutes sous 2,5 mA.cm².

Les réactions de marquage ont été réalisées à 37°C sous agitation modérée.

Les membranes sont incubées pendant 1 heure dans 100 ml de TBS-T contenant 5 % de gélatine, puis lavées avec du TBS-T (trois fois 15 minutes). Les membranes sont ensuite incubées séparément en présence de 20 ml de TBS-T additionné de lait écrémé à 1,2 % et de 2 µl du 1^{er} anticorps (sérum reconnaissant Sup 16) pendant 2 heures. Puis les membranes sont lavées dans 200 ml de TBS-T (trois fois 15 minutes), puis incubées pendant 1 heure dans 20 ml de TBS-T contenant 1,2 % de lait écrémé et 1 µl du second anticorps (anticorps de chèvre anti-lgG de lapin couplé à une peroxydase de radis, Bio-Rad). Les membranes sont ensuite lavées dans 200 ml de TBS-T (trois fois 15 minutes).

La révélation se fait au moyen du kit ECL « Western Blotting Detection Reagents » (Amersham), suivant les conditions recommandées par le fournisseur.

### EXEMPLE 4 : Activité anti-microbienne du peptide :

Des expériences de toxicité du peptide de 25 acides aminés (SUP 25) purifié à l'exemple précédent sur différentes bactéries ont été réalisées.

Les différentes bactéries sont cultivées dans 5ml de milieu LB ou de milieu approprié à la souche, puis sédimentées par centrifugation à 3000g, et enfin reprises dans un volume de LB identique au volume de bactéries. 50 µl de bactéries sont inoculés dans 9 ml de TOPagar (LB + 7g/l de bactoagar) liquide à 48°C et rapidement coulé sur une boite LB agar carrée de 10 cm de coté. Les gouttes de 10 µl de différentes concentrations de peptides sont déposées sur le TOPagar solidifié. La boite est mise en culture pendant 16 heures.

La cécropine A à 20 µM, peptide d'insecte reconnu comme ayant une activité antibactérienne, sert de témoin positif.

### Résultats :

| Bactéries | Pathogène de plante | Activité bactériostatique | Molarité active | Cécropine A 20 µM |
|---|---|---|---|---|
| *Escherichia coli* Souche DH5α | Non | Négatif | - | Positif |
| *Xanthomonas campestris* pv campestris souche 8004 | Oui | Positif | 200µM | Positif |
| *Pseudomonas syringae* pv tomato souche DC3000 | Oui | Positif | 20µM | Positif |
| *Erwinia amylovora* Souche 1430 | Oui | Positif | 25µM | Non testé |

Le peptide de 25 acides aminés ne présente pas d'activité sur *Escherichia coli,* mais présente une activité sur *Xanthomonas campestris, Pseudomonas syringae* et *Erwinia amylovora,* ce qui permet d'imaginer son utilisation comme agent anti-microbien particulièrement dirigé contre les agents pathogènes de plantes très particulièrement au moins contre *Xanthomonas campestris, Pseudomonas syringae* et/ou *Erwinia amylovora.*

### EXEMPLE 5 : Effet cytotoxique du peptide SUP 25 sur des protoplastes d'Arabidopsis thaliana :

Des essais de toxicité réalisés sur des cellules végétales, isolées, sans paroi, révèlent une perte de viabilité induite par l'addition de peptides synthétiques dans le milieu de culture. Certaines mutations de la séquence en acides aminés du peptide abolissent la toxicité.

### 5.1) Mesure de la viabilité par la coloration à la Fluorescéine DiAcétate (FDA) :

Les tests de viabilité par la coloration au FDA repose sur la détection de l'activité estérase spécifiquement présente dans les cellules vivantes. Le FDA pénètre dans la cellule, les résidus acétates sont coupés par l'activité estérase et la fluorescéine est libérée, conférant aux cellules vivantes une coloration verte sous excitation bleue. Quant aux cellules mortes, lorsqu'elles sont chlorophylliennes, elles fluorescent en rouge grâce aux pigments qu'elles contiennent.

Des protoplastes de feuilles *d'Arabidopsis thaliana* obtenus et mis en culture selon la méthode décrite par Danon et Gallois (FEBS letters, 1998, 437 : 131-136).

Le peptide synthétique de 25 acides aminés (SUP25) est ajouté aux protoplastes de feuilles *d'Arabidopsis thaliana* en culture.

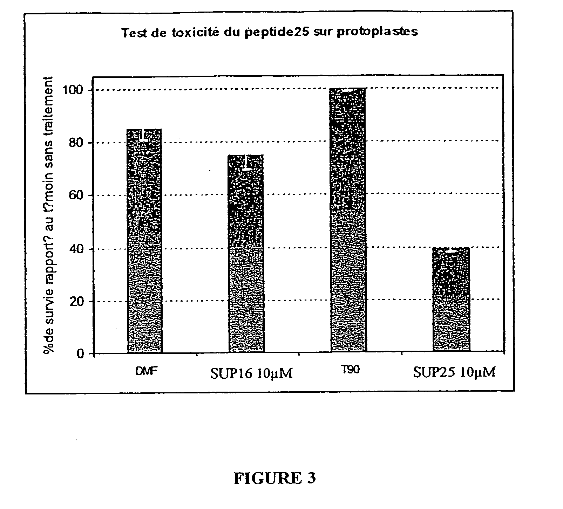

Une dose de 10 µM de SUP25 suffit à abaisser d'un facteur 2,5 la viabilité d'une population de cellules. (Figure 3).

En revanche le peptide tronqué de 16 acides aminés (pep 16) à 10 µM n'affecte le taux de protoplastes vivants que d'un facteur 1,3.

En raison de la forte hydrophobicité du peptide de 16 acides aminés, il a été utilisé dans du DiMethylFormamide (DMF) à 50%. Le témoin DMF seul affecte la viabilité d'un facteur 1,2. Ce qui permet de dire que l'effet du peptide tronqué sur la viabilité des protoplastes est très faible, voire nul, en comparaison de l'effet du peptide complet.

On peut donc conclure que la toxicité du peptide est spécifiquement liée aux 10 acides aminés de la région C-terminale du peptide SUP25.

D'autre part la dose impliquée dans l'effet correspond aux ordres de grandeur des doses létales (1 à 100 µM) de différents peptides anti-microbiens connus (Bulet P., Med. & Sc., 1999, 1, 23-9)

### 5.2) Détermination de la concentration optimale pour l'activité cytotoxique du peptide :

Différentes concentrations du peptide SUP25 (1 µM, 5 µM, 10 µMet 100 µM) ont été appliquées sur les protoplastes de feuilles *d'Arabidopsis thaliana* (Figure 4). A première vue, l'activité du peptide ne dépend pas de la dose, bien qu'un effet optimum soit observé à 5 µM. En raison de la procédure expérimentale et pour ne pas ajouter de trop grands volumes aux protoplastes, l'expérience a été menée en utilisant quatre solutions stocks aux concentrations de 1 mM, 5 mM, 10 mM et 100 mM respectivement.

Afin de contrôler l'homogénéité des stocks, une fraction aliquote, de même volume pour chaque solution de peptide, a été déposée sur gel de polyacrylamide en condition dénaturante (Figure 4, encart). A forte concentration, le peptide est impliqué dans la formation de complexes multimériques supérieurs à 30 kDa. La stabilité de ces complexes suggère l'intervention des cystéines dans la formation de ponts disulfures, seuls capables de résister au traitement SDS.

Cette expérience montre que la forme monomérique du peptide est la plus efficace et qu'une concentration de 5 µM du peptide conduit à une chute de viabilité très significative.

### 5.3) Mesure de la fragmentation de l'ADN suite aux traitements avec différents peptides modifiés :

Cette expérience a pour but de déterminer si l'effet du peptide sur la viabilité des protoplastes *d'Arabidopsis thaliana* correspond à de la nécrose cellulaire ou bien à la mise en place de la voie de la mort cellulaire programmée ou apoptose.

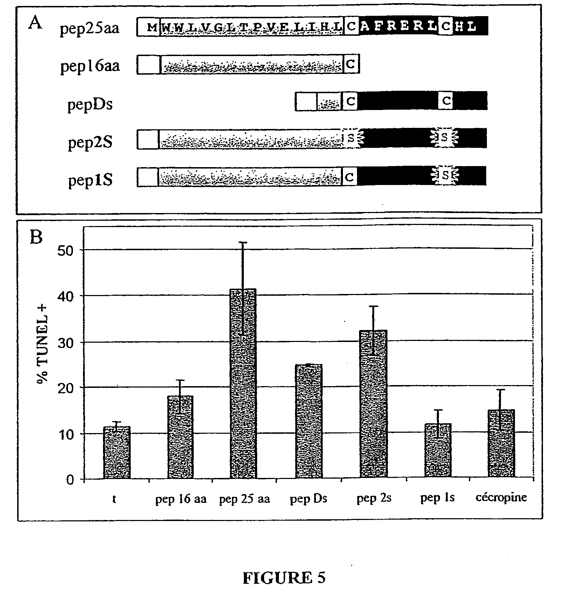

L'apoptose se distingue de la nécrose par une mise en scène rigoureuse de la mort, ponctuée par un ensemble d'événements génétiquement contrôlés (Vaux et Korsmeyer (Cell, 1999, 96 : 245-254) ; Pennell et Lamb (Plant Cell, 1997, 9, : 1157-1168) ; Greenberg (P.N.A.S., 1996, 93 :12094-12097)). L'une de ces étapes est la fragmentation de l'ADN nucléaire qui peut être mise en évidence par la technique TUNEL ("Terminal deoxynucleotidyl transferase-mediated dUTP Nick End Labeling") selon le protocole de Negoescu (Negoescu et al., Plant J., 1997, 13 : 803-814). La réaction de TUNEL permet de marquer les extrémités 3'OH libres présentes en grande quantité dans les noyaux dont l'ADN est fragmenté au cours du processus d'apoptose. La figure 5 présente les résultats de marquage TUNEL sur des protoplastes soumis à différents variants du peptide.

L'application externe du peptide de 25 acides aminés sur protoplastes *d'Arabidopsis thaliana* à 5 µM pendant 2 heures permet d'observer une augmentation de la population de protoplastes TUNEL positifs (figure 5B). 4 fois plus de noyaux présentent des caractéristiques de fragmentation d'ADN par rapport au témoin. Le peptide tronqué de 16 acides aminés (pep16) n'induit pas d'effet.

### 5.4) Etude de l'activité biologique de peptides modifiés au niveau des cystéines :

### 5.4.1) Construction et synthèse des peptides modifiés :

Afin d'étudier l'impact des cystéines sur l'activité biologique observée, les 3 peptides modifiés pepDs, Pep1S et Pep2S ont été synthétisés (Figure 5A) par la société Eurogenetec.

### 5.4.2) Activité cytotoxique des peptides modifiés :

La fragmentation de l'ADN nucléaire induite par ces peptides modifiés est mise en évidence par la technique TUNEL (figure 5B).

pepDs présente une activité résiduelle.

pep1S ne présente plus d'activité cytotoxique.

Pep2S présente une activité cytotoxique.

Ce résultat suggère que les cystéines sont impliquées dans la formation d'un pont disulfure intramoléculaire permettant ainsi au peptide de rester libre et actif.

### EXEMPLE 6 : Obtention de plantes transgéniques exprimant le peptide SUP25.

La séquence du gène *SUP25* peut être introduite dans une cassette d'expression utilisant divers promoteurs pour contrôler au niveau transcriptionnel, la production du peptide dans différents types de cellules végétales.

### 6.1) Synthèse de la séquence du gène :

En raison de la petite taille de l'ORF, la séquence codant le peptide SUP25 peut être synthétisée. Une paire d'oligonucléotides sens et antisens correspondant à la séquence du peptide est synthétisée à façon. La séquence est conçue de façon à ce que l'hybridation de la paire d'oligonucléotides laisse des extrémités cohésives correspondant à des sites de restrictions *Bam*HI en 5' et *Pst*I en 3' du fragment d'ADN. Les sites *Bam*HI/*Pst*I permettent de cloner le gène dans la cassette d'expression du plasmide pDH51 (Pietrzak M. et al., Expression in plants of two bacterial antibiotic résistance genes after protoplast transformation with a new plant expression vector. Nucleic Acids Res. 1986,14(14) : 5857-68) dans lequel le promoteur CaMV 35S et la région 3' du 35S dirige l'expression du gène introduit.

### 6.2) Ligation de la séquence du peptide dans une cassette d'expression

Les oligonucléotides sont hybridés à 1pmol/µl dans 10µl de tampon tfl 1X (Sambrook J. et al., Molecular Cloning : A Laboratory Manual. Ed. CSHLP, 2001) et sont incubés 1min à 95°C puis 10min à 45°C. Ensuite 20ng d'oligonudéotides (2µl) sont ligués dans 100 ng de vecteur pDH51 en utilisant les sites BamHI/PstI (Sambrook et al., 2001). Le produit de ligation est transformé dans *E.coli* XL1-B (Stratagène) en utilisant une méthode chimique (Sambrook et al., 2001) Les transformants sont sélectionnés sur l'antibiotique ampicilline. La présence de l'insert peut être directement identifiée dans les transformants par l'absence de couleur bleue sur milieu contenant le substrat X-GAL (Sambrook et al., 2001). Cinq transformants indépendants sont utilisés pour extraire le plasmide avec le kit commercial High pure plasmid Isolation kit (Roche/Boehringer). La présence de l'insert est vérifiée par restriction avec l'enzyme *Eco*RI (Sambrook et al, 2001). La fidélité de séquence des constructions est vérifiée pour la présence de la séquence SUP25 par séquençage (Sambrook et al., 2001) en utilisant une amorce appariant le promoteur 35S [op35S52 : CTTCGTCAACATGGTGGAGC] : (SEQ ID NO : 30) ou le terminateur 35|S [oter35 : CTAGCTAGAGGATCGATCC] : (SEQ ID NO : 31).

### 6.3) Transfert du transgène dans un vecteur binaire de transformation.

Le transgène est ensuite sous-cloné dans le site *Eco*RI de la région T-DNA du plasmide binaire pZP111 (Hajdukiewicz P, et al., The small, versatile pPZP family of Agrobacterium binary vectors for plant transformation. Plant Mol Biol., 1994, (6) : 989-94.), contenant un gène de sélection *NPTII* conférant aux plantes une résistance à l'antibiotique kanamycine. Le plasmide contient également un gène de sélection aadA pour la sélection chloramphénicol dans *E.coli* ou *Agrobacterium.* Le produit de ligation est transformé dans *E.coli* XL1-B (Stratagène) en utilisant une méthode chimique (Sambrook et al., 2001). Les transformants sont sélectionnés sur milieu LB (Sambrook et al., 2001) + 25mg/l chloramphénicol. La présence de l'insert dans le plasmide pZP111 peut être directement identifiée dans les transformants bactériens par l'absence de couleur bleue sur milieu contenant le substrat X-GAL (Sambrook et al., 2001).

### 6.4) Transfert du vecteur binaire dans Agrobacterium.

Une souche positive est sélectionnée, le plasmide est purifié en utilisant le kit commercial High pure plasmid Isolation kit (Roche/Boehringer). La souche désarmée *d'Agrobacterium tumefaciens* C58C1 (Koncz C. et Schell, J., The promoter of TI-DNA gene 5 controls the tissue-specific expression of chaemeric genes carried by a novel type of Agrobacterium binary vector, Mol. Gen. Genet., 204 : 383-396) est rendue compétente puis transformée par électroporation (Shen WJ. et Forde BG., Efficient transformation of Agrobacterium spp. by high voltage electroporation. Nucleic Acids Res. 1989, 17(20) : 8385) avec le plasmide binaire contenant la construction d'ADN-T. Après 3 jours de culture à 28°C, les colonies résistantes au chloramphénicol 25 mg/l, Rifampicine 50 mg/l, Gentamycine 25 mg/l, sont confirmées positives par PCR utilisant des amorces détectant le promoteur 35S [op35S52 : CTTCGTCAACATGGTGGAGC] (SEQ ID NO : 30) et le terminateur 35|S [oter35 : CTAGCTAGAGGATCGATCC] (SEQ ID NO : 31) (Sambrook et al 2001).

### 6.5) Obtention de plantes transgéniques.

Les plantes transgéniques sont ensuite obtenues par agroinfection en utilisant la souche C58C1 (Koncz et Schell, 1986) et la méthode de transformation de boutons floraux *d'Arabidopsis thaliana* selon le protocole décrit par Bechtold, N. et al., (In planta *Agrobacterium* mediated gene transfer by infiltration of adult *Arabidopsis thaliana* plants, C.R. Acad. Sci. (1993) Paris, 316, 1194). Brièvement, une préculture de 10 ml de YEP (extrait de Levure 10 g/l ; peptone 10 g/l ; glucose 20 g/l ; kanamycine 50 mg/l; rifampicine 50 mg/l ; gentamycine 25 mg/l est inoculée avec une colonie *d'Agrobacterium.* Après 16h sous agitation à 28°C, 500 ml de YEP sont ensemencés. Lorsque ,la culture atteint une DO₆₀₀ = 0,8, les bactéries sont récoltées après centrifugation à 3000g et mises en solution dans le milieu d'infiltration (MS medium 2,16 g/l (Duchefa) ; MES (Sigma) 0,5 g/l ; saccharose 5% ; pH 5,7 ajusté avec KOH ; Silwet L-77 (Osi Specialises Ins.) 0,02%.) à une DO finale de 0,5.

Les plantes *d'Arabidopsis thaliana,* au stade où les hampes florales commencent à initier les premières fleurs, sont trempées dans la solution d'infiltration pendant 10 minutes sous vide (-1 atm) dans un dessiccateur. Les plantes sont ensuite égouttées puis cultivées jusqu'à la récolte des graines avec les 2 premiers jours de culture sous film plastique. Les graines sont récoltées 4 semaines après l'infiltration. Après un minimum de 15 jours de stockage, les graines sont stérilisées par une solution d'éthanol à 70%, 10 min puis sélectionnées *in vitro* sur milieu de germination Gamborg B5 (Duchefa) 1,85 g/l et Phytagel (Sigma) 3 g/l contenant 400 mg/l de l'antibiotique kanamycine dont la résistance est portée par l'ADN-T. Les plantes résistantes (environ 1 % des graines) sont transplantées en terre, conservées sous plastique pendant 4 jours et cultivées jusqu'à la récolte des graines.

L'analyse du nombre de loci d'insertion du transgène se fait par test statistique de la ségrégation mendélienne du gène de sélection, ici pour la kanamycine, *in vitro* sur milieu de germination Gamborg B5 (Duchefa) 1,85 g/l et Phytagel (Sigma) 3 g/l contenant 400 mg/l de l'antibiotique kanamycine. Dix plantes avec un seul locus d'insertion sont sélectionnées (ségrégation 3:1) La présence du transgène est confirmée dans environ 8 plantes sur 10 par analyse PCR d'ADN purifié des plantes par la méthode (Dellaporta et al., Plant. Mol Biol. Rept., 1983, 1, 19-21) et utilisant des amorces détectant le promoteur 35S [op35S52 : CTTCGTCAACATGGTGGAGC] (SEQ ID NO : 30) et le terminateur 351S [oter35 : CTAGCTAGAGGATCGATCC] (SEQ ID NO : 31). L'expression du peptide est confirmée par analyse en western (Sambrook et al., 2001) d'extrait protéique des plantes transgéniques en utilisant un anticorps contre la séquence totale du peptide SUP25 sauvage et le kit de détection ECL (Amersham).

## Revendications

1. Peptide isolé, naturel ou synthétique, **caractérisé en ce qu'**il comprend au moins une séquence choisie parmi :
- les séquences SEQ ID NO : 1 à SEQ ID NO : 7, dans lesquelles l'acide aminé en position 16 est une cystéine ou une sérine, et
- les séquences SEQ ID NO : 8 à SEQ ID NO : 10, dans lesquelles l'acide aminé en position 16 et l'acide aminé en position 23 sont, indépendamment ou simultanément, une cystéine ou une sérine.

2. Peptide selon la revendication 1, **caractérisé en ce qu'**il consiste en l'une quelconque des séquences SEQ ID NO : 1 à SEQ ID NO : 10.

3. Peptide isolé, naturel ou synthétique, **caractérisé en ce que** sa séquence présente au moins 60% d'identité avec l'une quelconque des séquences SEQ ID NO : 1 à SEQ ID NO : 7, dans lesquelles l'acide aminé en position 16 est une cystéine ou une sérine, et SEQ ID NO : 8 à SEQ ID NO : 10, dans lesquelles l'acide aminé en position 16 et l'acide aminé en position 23 sont, indépendamment ou simultanément, une cystéine ou une sérine, et **en ce qu'**il conserve une activité anti-microbienne.

4. Peptide selon l'une quelconque des revendications 1 à 3, dont au moins un groupement fonctionnel libre, amine et/ou carboxylique, est protégé par un groupement protecteur.

5. Peptide selon la revendication 4, **caractérisé en ce que** le groupement carboxylique C-terminal et/ou les autres groupements carboxyliques présents dans la molécule sont sous la forme d'un ester ou d'un amide.

6. Peptide selon la revendication 4, **caractérisé en ce que** le groupement amine N-terminal, et/ou les autres groupements amines libres présents dans la molécule, sont sous forme acylée.

7. Composition comprenant au moins un peptide tel que décrit dans l'une quelconque des revendications 1 à 6 et au moins un véhicule approprié.

8. Utilisation d'au moins un peptide tel que décrit dans l'une quelconque des revendications 1 à 6, pour la préparation d'une composition anti-microbienne, dirigée contre les agents pathogènes de plantes.

9. Utilisation selon la revendication 8, **caractérisée en ce que** la composition anti-microbienne est dirigée contre *Xanthomonas campestris, Pseudomonas syringae* ou *d'Erwinia amylovora.*

10. Utilisation d'au moins un peptide comprenant ou consistant en au moins une séquence choisie parmi :
- les séquences SEQ ID NO : 2 à SEQ ID NO : 7, dans lesquelles l'acide aminé en position 16 est une cystéine ou une sérine, et
- les séquences SEQ ID NO : 8 à SEQ ID NO : 10, dans lesquelles l'acide aminé en position 16 est une cystéine ou une sérine et l'acide aminé en position 23 est une cystéine,
pour la préparation d'une composition cytotoxique, particulièrement pour les cellules végétales.

11. Utilisation d'au moins un peptide comprenant ou consistant en au moins l'une quelconque des séquences SEQ ID NO : 8 à SEQ ID NO : 10, dans lesquelles les acides aminés en positions 16 et 23 sont deux sérines, pour la préparation d'une composition cytotoxique, particulièrement d'une composition cytotoxique pour les cellules végétales.

12. Utilisation selon l'une quelconque des revendications 10 ou 11, **caractérisée en ce que** ladite composition est une composition désherbante.

13. Utilisation d'au moins un peptide comprenant ou consistant en au moins l'une quelconque des séquences SEQ ID NO : 8 à SEQ ID NO : 10, dans lesquelles l'acide aminé en position 16 est une cystéine et l'acide aminé en position 23 est une sérine, pour la préparation d'une composition anti-microbienne non cytotoxique, particulièrement d'une composition anti-microbienne non cytotoxique dirigée contre les agents pathogènes de plantes.

14. Polynucléotide isolé, naturel ou synthétique, **caractérisé en ce qu'**il consiste en une séquence codante pour l'un au moins des peptides selon l'une quelconque des revendications 1 à 6.

15. Polynucléotide selon la revendication 14, **caractérisé en ce qu'**il consiste en une séquence choisie parmi :
- les séquences SEQ ID NO : 11 à SEQ ID NO : 17, dans lesquelles le triplet de nucléotides 46 à 48 forme un codon cystéine ou un codon sérine, et
- les séquences SEQ ID NO : 18 à SEQ ID NO : 20, dans lesquelles le triplet de nucléotides 46 à 48 et le triplet de nucléotides 67 à 69 forment un codon cystéine ou un codon sérine.

16. Composition comprenant au moins un polynucléotide tel que décrit dans l'une quelconque des revendications 14 ou 15 et au moins un véhicule approprié.

17. Vecteur comprenant au moins l'un quelconque des polynucléotides décrits aux revendications 14 ou 15.

18. Vecteur selon la revendication 17, **caractérisé en ce qu'**il s'agit d'un vecteur plasmidique.

19. Utilisation d'un polynucléotide selon l'une quelconque des revendications 14 ou 15 ou d'un vecteur tel que décrit dans l'une quelconque des revendications 17 ou 18 pour la préparation d'un peptide tel que décrit dans l'une quelconque des revendications 1 à 6.

20. Système biologique modifié, **caractérisé en ce qu'**il est constitué par un microorganisme, un endophyte, une levure ou une cellule eucaryote et **en ce qu'**il contient au moins l'un des vecteurs tels que décrits dans l'une quelconque des revendications 17 ou 18, et/ou au moins l'un des polynucléotides tels que décrits dans l'une quelconque des revendications 14 ou 15.

21. Composition comprenant au moins un système biologique tel que décrit dans la revendication 20 et au moins un véhicule approprié.

22. Utilisation d'au moins un système biologique tel que décrit dans la revendication 20 ou d'une composition telle que décrite à la revendication 21, pour la préparation d'une composition anti-microbienne, particulièrement d'une composition anti-microbienne dirigée contre les agents pathogènes de plantes et/ou pour la préparation d'une composition cytotoxique.

23. Utilisation d'au moins un polynucléotide tel que décrit dans l'une quelconque des revendications 14 ou 15, ou d'au moins un vecteur tel que décrit dans l'une quelconque des revendications 17 ou 18, dans la préparation d'un système biologique modifié constitué par un microorganisme, un endophyte, une levure ou une cellule eucaryote.

24. Organisme transgénique non humain, **caractérisé en ce que** tout ou partie de ses cellules contient au moins un polynucléotide tel que décrit dans l'une quelconque des revendications 14 ou 15, ou au moins un vecteur tel que décrit dans l'une quelconque des revendications 17 ou 18.

25. Organisme transgénique selon la revendication 24, **caractérisé en ce qu'**il s'agit d'un végétal.

26. Anticorps, polyclonal ou monoclonal, dirigé contre au moins l'un des peptides tels que décrits dans l'une quelconque des revendications 1 à 6.

27. Procédé de traitement anti-microbien et/ou cytotoxique des végétaux, dans lequel on met en contact par tout moyen approprié un organisme, particulièrement un végétal, et au moins un agent anti-microbien, et/ou cytotoxique choisi parmi au moins l'un des peptides tels que décrits dans l'une quelconque des revendications 1 à 6, et/ou l'un des vecteurs tels que décrits dans l'une quelconque des revendications 17 ou 18, et/ou les polynucléotides tels que décrits dans l'une quelconque des revendications 14 ou 15, et/ou l'un des systèmes biologiques modifiés tels que décrits dans la revendication 20 et/ou un organisme transgénique, tel que décrit dans l'une quelconque des revendications 24 ou 25 ou les compositions telles que décrites dans l'une quelconque des revendications 7, 16 ou 21.

## Claims

1. Isolated natural or synthetic peptide, **characterised in that** it comprises at least one sequence selected from among:
- the sequences SEQ ID NO. 1 to SEQ ID NO. 7, wherein the amino acid in position 16 is a cysteine or a serine, and
- the sequences SEQ ID NO. 8 to SEQ ID NO. 10, wherein the amino acid in position 16 and the amino acid in position 23 are, independently or simultaneously, a cysteine or a serine.

2. Peptide according to claim 1, **characterised in that** it consists of any one of the sequences SEQ ID NO. 1 to SEQ ID NO. 10.

3. Isolated natural or synthetic peptide, **characterised in that** its sequence has at least 60% identity with any one of the sequences SEQ ID NO. 1 to SEQ ID NO. 7, wherein the amino acid in position 16 is a cysteine or a serine, and sequences SEQ ID NO. 8 to SEQ ID NO. 10, wherein the amino acid in position 16 and the amino acid in position 23 are, independently or simultaneously, a cysteine or a serine, and **in that** it retains an anti-microbial activity.

4. Peptide according to any one of claims 1 to 3, wherein at least one free functional amino and/or carboxyl group is protected by a protecting group.

5. Peptide according to claim 4, **characterised in that** the C-terminal carboxyl group and/or the other carboxyl groups present in the molecule are in the form of an ester or an amide.

6. Peptide according to claim 4, **characterised in that** the N-terminal amine group and/or the other free amine groups present in the molecule are in acylated form.

7. Composition comprising at least one peptide as described in any one of claims 1 to 6 and at least one suitable carrier.

8. Use of at least one peptide as described in any one of claims 1 to 6, for preparing an anti-microbial composition directed against plant pathogens.

9. Use according to claim 8, **characterised in that** the anti-microbial composition is directed against *Xanthomonas campestris, Pseudomonas syringae* or *Erwinia amylovora.*

10. Use of at least one peptide comprising or consisting of at least one sequence selected from among:
- the sequences SEQ ID NO. 2 to SEQ ID NO. 7, wherein the amino acid in position 16 is a cysteine or a serine, and
- the sequences SEQ ID NO. 8 to SEQ ID NO. 10, wherein the amino acid in position 16 is a cysteine or a serine and the amino acid at position 23 is a cysteine, for the preparation of a cytotoxic composition, particularly for plant cells.

11. Use of at least one peptide comprising or consisting of at least one of the sequences SEQ ID NO. 8 to SEQ ID NO. 10, wherein the amino acids in positions 16 and 23 are two serines, for the preparation of a cytotoxic composition, particularly for plant cells.

12. Use according to any one of claims 10 or 11, **characterised in that** the said composition is a weedkiller composition.

13. Use of at least one peptide comprising or consisting of at least one of the sequences SEQ ID NO. 8 to SEQ ID NO. 10, wherein the amino acid in position 16 is a cysteine and the amino acid in position 23 is a serine, for the preparation of a non-cytotoxic antimicrobial composition, particularly a non-cytotoxic antimicrobial composition directed against plant pathogens.

14. Isolated natural or synthetic polynucleotide, **characterised in that** it consists of a sequence coding for at least one of the peptides according to any one of claims 1 to 6.

15. Polynucleotide according to claim 14, **characterised in that** it consists of a sequence selected from among:
- the sequences SEQ ID NO. 11 to SEQ ID NO. 17, wherein the triplet of nucleotides 46 to 48 forms a cysteine codon or a serine codon, and
- the sequences SEQ ID NO. 18 to SEQ ID NO. 20, wherein the triplet of nucleotides 46 to 48 and the triplet of nucleotides 67 to 69 forms a cysteine codon or a serine codon.

16. Composition comprising at least one polynucleotide as described in any one of claims 14 or 15 and at least one suitable carrier.

17. Vector comprising at least one of the polynucleotides described in claims 14 or 15.

18. Vector according to claim 17, **characterised in that** it is a plasmid vector.

19. Use of a polynucleotide according to any one of claims 14 or 15 or of a vector as described in any one of claims 17 or 18 for preparing a peptide as described in any one of claims 1 to 6.

20. Modified biological system, **characterised in that** it consists of a micro-organism, an endophyte, a yeast or a eukaryotic cell and **in that** it contains at least one of the vectors as described in any one of claims 17 or 18, and/or at least one of the polynucleotides as described in any one of claims 14 or 15.

21. Composition comprising at least one biological system as described in claim 20 and at least one suitable carrier.

22. Use of at least one biological system as described in claim 20 or of a composition as described in claim 21, for the preparation of an antimicrobial composition, particularly an antimicrobial composition directed against plant pathogens and/or for the preparation of a cytotoxic composition.

23. Use of at least one polynucleotide as described in any one of claims 14 or 15, or of at least one vector as described in any one of claims 17 or 18, in the preparation of a modified biological system constituted by a micro-organism, an endophyte, a yeast or a eukaryotic cell.

24. Non-human transgenic organism, **characterised in that** all or some of its cells contain at least one polynucleotide as described in any one of claims 14 or 15, or at least one vector as described in any one of claims 17 or 18.

25. Transgenic organism according to claim 24, **characterised in that** it is a plant.

26. Polyclonal or monoclonal antibody, directed against at least one of the peptides as described in any one of claims 1 to 6.

27. Process for antimicrobial and/or cytotoxic treatment of plants, wherein an organism, particularly a plant, is brought into contact by any suitable means with at least one antimicrobial and/or cytotoxic agent selected from among at least one of the peptides as described in any one of claims 1 to 6, and/or one of the vectors as described in any one of claims 17 or 18, and/or the polynucleotides as described in any one of claims 14 or 15, and/or one of the modified biological systems as described in claim 20 and/or a transgenic organism, as described in any one of claims 24 or 25 or the compositions as described in any one of claims 7, 16 or 21.

## Patentansprüche

1. Natürliches oder synthetisches isoliertes Peptid, **dadurch gekennzeichnet, dass** es wenigstens eine Sequenz umfasst, die ausgewählt ist aus:
- den Sequenzen SEQ ID NO: 1 bis SEQ ID NO: 7, in denen die Aminosäure in Position 16 ein Cystein oder ein Serin ist, und
- den Sequenzen SEQ ID NO: 8 bis SEQ ID NO: 10, in denen die Aminosäure in Position 16 und die Aminosäure in Position 23 unabhängig oder gleichzeitig ein Cystein oder ein Serin sind.

2. Peptid nach Anspruch 1, **dadurch gekennzeichnet, dass** es aus einer der Sequenzen SEQ ID NO: 1 bis SEQ ID NO: 10 besteht.

3. Natürliches oder synthetisches isoliertes Peptid, **dadurch gekennzeichnet, dass** seine Sequenz wenigstens 60% Identität mit einer der Sequenzen von SEQ ID NO: 1 bis SEQ ID NO: 7, in denen die Aminosäure in Position 16 ein Cystein oder ein Serin ist, und SEQ ID NO: 8 bis SEQ ID NO: 10, in denen die Aminosäure in Position 16 und die Aminosäure in Position 23 unabhängig oder gleichzeitig ein Cystein oder ein Serin sind, aufweist und dass es eine antimikrobielle Aktivität konserviert.

4. Peptid nach einem der Ansprüche 1 bis 3, bei dem wenigstens eine freie funktionelle Gruppe, Amino- und/oder Carboxylgruppe, durch eine Schutzgruppe geschützt ist.

5. Peptid nach Anspruch 4, **dadurch gekennzeichnet, dass** die C-terminale Carboxylgruppe und/oder die anderen Carboxylgruppen, die in dem Molekül vorliegen, in der Form eines Esters oder eines Amids sind.

6. Peptid nach Anspruch 4, **dadurch gekennzeichnet, dass** die N-terminale Aminogruppe und/oder die anderen freien Aminogruppen, die in dem Molekül vorliegen, in acylierter Form sind.

7. Zusammensetzung, umfassend wenigstens ein Peptid, wie es in einem der Ansprüche 1 bis 6 beschrieben ist, und wenigstens ein geeignetes Vehikel.

8. Verwendung wenigstens eines Peptids, wie es in einem der Ansprüche 1 bis 6 beschrieben ist, für die Herstellung einer antimikrobiellen Zusammensetzung, die gegen die Krankheitserreger von Pflanzen gerichtet ist.

9. Verwendung nach Anspruch 8, **dadurch gekennzeichnet, dass** die antimikrobielle Zusammensetzung gegen *Xanthomonas campestris, Pseudomonas syringae* oder *Erwinia amylovora* gerichtet ist.

10. Verwendung wenigstens eines Peptids, umfassend wenigstens eine Sequenz oder bestehend aus wenigstens einer Sequenz, ausgewählt aus:
- den Sequenzen SEQ ID NO: 1 bis SEQ ID NO: 7, in denen die Aminosäure in Position 16 ein Cystein oder ein Serin ist, und
- den Sequenzen SEQ ID NO: 8 bis SEQ ID NO: 10, in denen die Aminosäure in Position 16 und die Aminosäure in Position 23 unabhängig oder gleichzeitig ein Cystein oder ein Serin sind,
für die Herstellung einer insbesondere für Pflanzenzellen cytotoxischen Zusammensetzung.

11. Verwendung wenigstens eines Peptids, das wenigstens eine der Sequenzen SEQ ID NO: 8 bis SEQ ID NO: 10, in denen die Aminosäuren in Positionen 16 und 23 zwei Serine sind, umfasst oder aus wenigstens einer dieser Sequenzen besteht, für die Herstellung einer cytotoxischen Zusammensetzung, insbesondere einer für Pflanzenzellen cytotoxischen Zusammensetzung.

12. Verwendung nach einem der Ansprüche 10 oder 11, **dadurch gekennzeichnet, dass** die Zusammensetzung eine Herbizid-Zusammensetzung ist.

13. Verwendung wenigstens eines Peptids, das wenigstens eine der Sequenzen SEQ ID NO: 8 bis SEQ ID NO: 10, in denen die Aminosäure in Position 16 ein Cystein ist und die Aminosäure in Position 23 ein Serin ist, umfasst oder aus einer dieser Sequenzen besteht, für die Herstellung einer nicht cytotoxischen antimikrobiellen Zusammensetzung, insbesondere einer nicht cytotoxischen antimikrobiellen Zusammensetzung, die gegen die Krankheitserreger von Pflanzen gerichtet ist.

14. Natürliches oder synthetisches isoliertes Polynucleotid, **dadurch gekennzeichnet, dass** es aus einer Sequenz besteht, die für wenigstens eines der Peptide nach einem der Ansprüche 1 bis 6 codiert.

15. Polynucleotid nach Anspruch 14, **dadurch gekennzeichnet, dass** es aus einer Sequenz besteht, die ausgewählt ist aus:
- den Sequenzen SEQ ID NO: 11 bis SEQ ID NO: 17, in denen das Triplett der Nucleotide 46 bis 48 ein Cysteincodon oder ein Serincodon bildet, und
- den Sequenzen SEQ ID NO: 18 bis SEQ ID NO: 20, in denen das Triplett der Nucleotide 46 bis 48 und das Triplett der Nucleotide 67 bis 69 ein Cysteincodon oder ein Serincodon bilden.

16. Zusammensetzung, umfassend wenigstens ein Nucleotid, wie es in einem der Ansprüche 14 oder 15 beschrieben ist, und wenigstens ein geeignetes Vehikel.

17. Vektor, der wenigstens eines der Polynucleotide, die in den Ansprüchen 14 oder 15 beschrieben sind, umfasst.

18. Vektor nach Anspruch 17, **dadurch gekennzeichnet, dass** es sich um einen Plasmidvektor handelt.

19. Verwendung eines Polynucleotids nach einem der Ansprüche 14 oder 15 oder eines Vektors, wie er in einem der Ansprüche 17 oder 18 beschrieben ist, für die Herstellung eines Peptids, wie es in einem der Ansprüche 1 bis 6 beschrieben ist.

20. Modifiziertes biologisches System, **dadurch gekennzeichnet, dass** es durch einen Mikroorganismus, einen Endophyten, eine Hefe oder eine Eukaryotenzelle gebildet wird und dass es wenigstens einen der Vektoren, wie er in einem der Ansprüche 17 oder 18 beschrieben ist, und/oder wenigstens eines der Polynucleotide, wie es in einem der Ansprüche 14 oder 15 beschrieben ist, enthält.

21. Zusammensetzung, die wenigstens ein biologisches System, wie es in Anspruch 20 beschrieben ist, und wenigstens ein geeignetes Vehikel umfasst.

22. Verwendung wenigstens eines biologischen Systems, wie es in Anspruch 20 beschrieben ist, oder einer Zusammensetzung, wie sie in Anspruch 21 beschrieben ist, für die Herstellung einer antimikrobiellen Zusammensetzung, insbesondere einer antimikrobiellen Zusammensetzung, die gegen die Krankheitserreger von Pflanzen gerichtet ist, und/oder für die Herstellung einer cytotoxischen Zusammensetzung.

23. Verwendung wenigstens eines Polynucleotids, wie es in einem der Ansprüche 14 oder 15 beschrieben ist, oder wenigstens eines Vektors, wie er in einem der Ansprüche 17 oder 18 beschrieben ist, bei der Herstellung eines modifizierten biologischen Systems, das durch einen Mikroorganismus, einen Endophyten, eine Hefe oder eine Eukaryotenzellen gebildet wird.

24. Nichtmenschlicher transgener Organismus, **dadurch gekennzeichnet, dass** die Gesamtheit oder ein Teil seiner Zellen wenigstens ein Polynucleotid, wie es in einem der Ansprüche 14 oder 15 beschrieben ist, oder wenigstens einen Vektor, wie er in einem der Ansprüche 17 oder 18 beschrieben ist, enthält.

25. Transgener Organismus nach Anspruch 24, **dadurch gekennzeichnet, dass** es sich um eine Pflanze handelt.

26. Polyclonaler oder monoclonaler Antikörper, der gegen wenigstens eines der Peptide, wie sie in einem der Ansprüche 1 bis 6 beschrieben sind, gerichtet ist.

27. Verfahren zur antimikrobiellen und/oder cytotoxischen Behandlung von Pflanzen, in dem man durch jedes geeignete Mittel einen Organismus, insbesondere eine Pflanze, und wenigstens ein antimikrobielles und/oder cytotoxisches Mittel, ausgewählt aus wenigstens einem der Peptide, wie sie in einem der Ansprüche 1 bis 6 beschrieben sind, und/oder einem der Vektoren, wie sie in einem der Ansprüche 17 oder 18 beschrieben sind, und/oder den Polynucleotiden, wie sie in einem der Ansprüche 14 oder 15 beschrieben sind, und/oder einem der modifizierten biologischen Systeme, wie sie in Anspruch 20 beschrieben sind, und/oder einem transgenen Organismus, wie er in einem der Ansprüche 24 oder 25 beschrieben ist, und den Zusammensetzungen, wie sie in einem der Ansprüche 7, 16 oder 21 beschrieben sind, miteinander in Kontakt bringt.
